# EUROPEAN PATENT APPLICATION

(11) **EP 4 086 274 A1**
(43) Date of publication of application: **09.11.2022**
(21) Application number: 20909593.4
(22) Date of filing: 25.12.2020
(51) Int. Cl.: C07K 14/435, C07K 14/00, G01N 33/68, G01N 33/574, G01N 33/569

(54) **POLYPEPTIDE ENCODED BY BNLF2B GENE IN EB VIRUS AND DETECTION USE THEREOF**

(30) Priority: 02.01.2020 CN 202010001204
(71) Applicant: Xiamen University, Xiamen, Fujian 361005 (CN); Xiamen Innodx Biotech Co., Ltd, Xiamen, Fujian 361002 (CN)
(72) Inventor: LI, Tingdong, Xiamen, Fujian 361102 (CN); GE, Shengxiang, Xiamen, Fujian 361102 (CN); GUO, Xiaoyi, xiamen, fujian 361102 (CN); HONG, Congming, Xiamen, Fujian 361102 (CN); SONG, Liuwei, Xiamen, Fujian 361022 (CN); WEN, Shunhua, Xiamen, Fujian 361022 (CN); TANG, Jiabao, Xiamen, Fujian 361102 (CN); ZHANG, Jun, Xiamen, Fujian 361102 (CN); XIA, Ningshao, Xiamen, Fujian 361102 (CN)
(74) Representative: Lavoix
(86) International application number: PCT/CN2020/139310
(87) International publication number: WO 2021/136082

(57) **Abstract**

Provided are a method for diagnosing nasopharyngeal carcinoma on the basis of the anti-EB virus (EBV) antibody level and a kit used for the method. Also provided are a polypeptide encoded by a BNLF2b gene in EB virus used for the above-mentioned diagnosis and the use thereof for diagnosing nasopharyngeal carcinoma.

## Description

### Technical Field

The present invention relates to immunological detection, in particular to the field of immunological diagnosis of tumors. Specifically, the present invention provides a method for diagnosing nasopharyngeal cancer based on level of anti-Epstein-Barr virus (EBV) antibody, and a kit for use in the method. The present invention also provides a polypeptide encoded by the EBV BNLF2b gene used for the above diagnosis and use thereof in the diagnosis of nasopharyngeal cancer.

### Background Art

Nasopharyngeal cancer is a malignant tumor that occurs in the top and lateral walls of the nasopharyngeal cavity, and its incidence ranks first among malignant tumors of ear, nose and throat. China is a high-incidence area of nasopharyngeal cancer, with the highest incidence in Guangdong and Guangxi [1]. The occurrence of nasopharyngeal cancer is related to many factors such as EBV infection, genetic factors, environmental factors [2]. Because the early symptoms of nasopharyngeal cancer are not obvious, and the incidence location is relatively hidden, most nasopharyngeal cancers cannot be detected until the advanced stage [3]. However, the five-year survival rate of early nasopharyngeal cancer can reach 90% or more, while the prognosis of advanced nasopharyngeal cancer is significantly lower [3]. Meanwhile, early nasopharyngeal cancer can be treated with local radiotherapy, while advanced nasopharyngeal cancer requires the treatment with chemotherapy. Therefore, early diagnosis is the key to improve the survival rate and life quality of nasopharyngeal cancer patients.

As early as 1976, Werner Henle et al found that EBV IgA antibody in serum in patients with nasopharyngeal cancer was significantly increased [4]. Subsequently, studies found that the levels of antibodies against EBNA1, EA-D, VCA and other proteins in the serum of patients with nasopharyngeal cancer were significantly increased [5-12]. Early EBV antibody detection was mainly based on immunofluorescence, which was low throughput, time-consuming and highly subjective, making it difficult to meet the needs of large-scale screening [13-15]; with the development of enzyme-linked immunosorbent assay (ELISA), researchers from various countries had established a large number of EBV antibody ELISA detection kits with significantly improved detection throughput and objectivity [16-23]. In addition, studies also found that the sensitivity and specificity of nasopharyngeal cancer screening could be further improved by combination of different serological markers, of which the combination of EBNA1-IgA and VCA-IgA was the most widely used [24-26]. The results of prospective cohort studies had shown that the early diagnosis rate of nasopharyngeal cancer could be increased from 10-20% to 60% or more through screening [27-29]. On this basis, our research group proposed a two-step screening strategy, that was, further detection of EAD-IgA was performed in the high-risk population identified in the first step, based on combined screening using EBNA1-IgA and VCA-IgA, and the EAD-IgA positive patients were confirmed by nasopharyngeal endoscopy. With the two-step screening, the positive predictive value of nasopharyngeal cancer screening was increased from 4.69% to 18.52% [30].

In addition to EBV antibody levels, the levels of EBV DNA and micro RNA in the blood of nasopharyngeal cancer patients were also significantly elevated [31-33]. The results of a prospective study conducted by Allan Chan et al in Hong Kong showed that using persistently positive result for EBV DNA as the standard for nasopharyngeal cancer screening could significantly improve the specificity of nasopharyngeal cancer screening; among the 1112 patients (5.5%) who were EBV DNA positive in the initial screening, only 309 patients were persistently positive for EBV DNA, and 34 of them were eventually diagnosed with nasopharyngeal cancer [34]. Taking persistently positive result for EBV as the screening standard, the sensitivity and specificity reached 97.1% and 98.6%, respectively, the positive predictive value increased from 3.06% to 11%, and the early diagnosis rate of nasopharyngeal cancer increased from 20% to 71% through screening [34].

Compared with one-step screening, the positive predictive value of the two-step screening is significantly improved, which can meet the needs of larger-scale nasopharyngeal cancer screening. However, the process for two-step screening is more complicated and costly for either serological screening or DNA screening. In addition, the persistent positive result for DNA requires further blood collection, which further increases the difficulty of screening. Therefore, although one-step method of serological antibody screening is significantly cost-effective [35], whether two-step screening is more cost-effective needs further evaluation. In addition, the highest positive predictive value of the currently reported method of nasopharyngeal cancer screening is only 18.52%, that is, at least 71.48% of non-nasopharyngeal cancer patients in the screened high-risk population should undergo nasopharyngeal endoscopy. However, nasopharyngeal endoscopy takes a long time, which directly limits the scale of nasopharyngeal cancer screening, and at the same time, it will also cause a psychological burden on non-nasopharyngeal cancer patients who are positive in the screening.

Therefore, there is still a need in the art to develop new screening method for nasopharyngeal cancer, which will have higher specificity and positive predictive value than existing methods, and has the characteristics of high throughput and low cost to meet the needs of large-scale screening, thereby increasing the number of people who can benefit from the screening.

### Contents of the present invention

After extensive research, the inventors of the present application unexpectedly found that a nasopharyngeal cancer patient can be efficiently distinguished from a healthy control based on the antibody level specific to a protein encoded by EBV BNLF2b gene in the serum of a subject (which can be, for example, detected by an indirect method or double antigen sandwich method using a protein or polypeptide fragment encoded by the BNLF2b gene as the antigen for coating). In addition, with considerable creative effort, the inventors obtained a polypeptide fragment encoded by the BNLF2b gene that is particularly suitable as a capture reagent (for example, an antigen for coating in ELISA). Thus, the inventors successfully establish a method and platform for diagnosing nasopharyngeal cancer based on detection of the antibody level against a protein encoded by EBV BNLF2b gene, which can significantly improve the specificity and positive predictive value of nasopharyngeal cancer screening.

### Isolated polypeptide or variant thereof

In a first aspect, the present invention provides an isolated polypeptide or variant thereof, wherein the polypeptide consists of at least 7 contiguous amino acid residues of a wild-type protein encoded by BNLF2b gene, and comprises at least 1 (e.g., at least 2, at least 3, or all 4) of the following sequences: amino acid residues 5-11, amino acid residues 16-23, amino acids 31-39, or amino acid residues 53-60 (e.g., amino acid residues 53-61) of a wild-type protein encoded by the BNLF2b gene;
wherein the variant differs from the polypeptide from which it is derived only by a substitution (e.g., conservative substitution or non-conservative substitution) of one or several (e.g., 1, 2, 3, 4, 5, 6, 7, 8, or 9) amino acid residues, and retains a biological function of the polypeptide from which it is derived (e.g., an activity of being recognized and bound by an anti-EBV antibody).

In certain embodiments, the wild-type protein encoded by the BNLF2b gene has the sequence set forth in SEQ ID NO: 101.

Herein, the biological function of the polypeptide or variant thereof of the present invention includes, but is not limited to, the activity of being recognized and bound by an anti-EBV antibody (e.g., an antibody specific for the protein encoded by the BNLF2b gene) as an epitope peptide.

In certain embodiments, the isolated polypeptide comprises: amino acid residues 53-60 (e.g., amino acid residues 53-61), amino acid residues 5-23, amino acid residues 16-39, amino acid residues 31-60 (e.g., amino acid residues 31-61), amino acid residues 5-39, amino acid residues 16-60 (e.g., amino acid residues 16-61), or amino acid residues 5-60 (e.g., amino acid residues 5-61) of the wild-type protein encoded by the BNLF2b gene.

In one embodiment, the isolated polypeptide consists of at least 8 (e.g., at least 9, at least 10, at least 11, at least 12, at least 13, at least 14, at least 14, or at least 15) contiguous amino acid residues of the wild-type protein encoded by the BNLF2b gene, and comprises amino acid residues 53-60 of the wild-type protein encoded by the BNLF2b gene.

In certain embodiments, the polypeptide comprises amino acid residues 53-61 of the wild-type protein encoded by the BNLF2b gene.

In certain embodiments, the polypeptide comprises amino acid residues 51-65 of the wild-type protein encoded by the BNLF2b gene.

In certain embodiments, the amino acid residues 51-65 have a sequence set forth in SEQ ID NO:97.

In certain embodiments, the polypeptide consists of at least 15 contiguous amino acid residues of the wild-type protein encoded by the BNLF2b gene.

In one embodiment, the isolated polypeptide consists of at least 19 (e.g., at least 20, at least 21, at least 22, at least 23, at least 24, at least 25) contiguous amino acid residues of the wild-type protein encoded by the BNLF2b gene, and comprises amino acid residues 5-23 of the wild-type protein encoded by the BNLF2b gene.

In certain embodiments, the polypeptide comprises amino acid residues 1-25 of the wild-type protein encoded by the BNLF2b gene.

In certain embodiments, the amino acid residues 1-25 have a sequence set forth in SEQ ID NO: 102.

In certain embodiments, the polypeptide consists of at least 25 contiguous amino acid residues of the wild-type protein encoded by the BNLF2b gene.

In one embodiment, the isolated polypeptide consists of at least 24 (e.g., at least 25, at least 26, at least 27, at least 28, at least 29, at least 30, at least 31, at least 32, at least 33, at least 34, at least 35, at least 36, at least 37, at least 38, or at least 39) contiguous amino acid residues of the wild-type protein encoded by the BNLF2b gene, and comprises amino acid residues 16-39 of the wild-type protein encoded by the BNLF2b gene.

In certain embodiments, the polypeptide comprises amino acid residues 14-52 of the wild-type protein encoded by the BNLF2b gene.

In certain embodiments, the amino acid residues 14-52 have the sequence set forth in SEQ ID NO:88.

In certain embodiments, the polypeptide consists of at least 39 contiguous amino acid residues of the wild-type protein encoded by the BNLF2b gene.

In one embodiment, the isolated polypeptide consists of at least 30 contiguous amino acid residues of the wild-type protein encoded by the BNLF2b gene, and comprises amino acid residues 31-60 of the wild-type protein encoded by the BNLF2b gene.

In certain embodiments, the isolated polypeptide comprises amino acid residues 31-61 of the wild-type protein encoded by the BNLF2b gene.

In one embodiment, the isolated polypeptide consists of at least 35 (e.g., at least 36, at least 37, at least 38, at least 39, at least 40, at least 41, at least 42, at least 43, at least 44, at least 45, at least 46, at least 47, at least 48, at least 49, at least 50, at least 51, at least 52, or at least 53) contiguous amino acid residues of the wild-type protein encoded by the BNLF2b gene, and comprises amino acid residues 5-39 of the wild-type protein encoded by the BNLF2b gene.

In certain embodiments, the polypeptide comprises amino acid residues 1-52 of the wild-type protein encoded by the BNLF2b gene.

In certain embodiments, the amino acid residues 1-52 have a sequence set forth in SEQ ID NO:91.

In certain embodiments, the polypeptide consists of at least 53 contiguous amino acid residues of the wild-type protein encoded by the BNLF2b gene.

In one embodiment, the isolated polypeptide consists of at least 45 (e.g., at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, or at least 64) contiguous amino acid residues of the wild-type protein encoded by the BNLF2b gene, and comprises amino acid residues 16-60 of the wild-type protein encoded by the BNLF2b gene.

In certain embodiments, the polypeptide comprises amino acid residues 16-61 of the wild-type protein encoded by the BNLF2b gene.

In certain embodiments, the polypeptide comprises amino acid residues 14-74 of the wild-type protein encoded by the BNLF2b gene (e.g., the sequence set forth in SEQ ID NO: 90). In certain embodiments, the polypeptide consists of at least 61 contiguous amino acid residues of the wild-type protein encoded by the BNLF2b gene.

In certain embodiments, the polypeptide comprises amino acid residues 11-65 of the wild-type protein encoded by the BNLF2b gene (e.g., the sequence set forth in SEQ ID NO: 103). In certain embodiments, the polypeptide consists of at least 55 contiguous amino acid residues of the wild-type protein encoded by the BNLF2b gene.

In certain embodiments, the polypeptide comprises amino acid residues 11-74 of the wild-type protein encoded by the BNLF2b gene (e.g., the sequence set forth in SEQ ID NO: 104). In certain embodiments, the polypeptide consists of at least 64 contiguous amino acid residues of the wild-type protein encoded by the BNLF2b gene.

In one embodiment, the isolated polypeptide consists of at least 56 (e.g., at least 57, at least 58, at least 59, at least 60, at least 61, at least 62, at least 63, at least 64, at least 65, at least 66, at least 67, at least 68, at least 69, at least 70, at least 71, at least 72, at least 73, or at least 74) contiguous amino acid residues of the wild-type protein encoded by the BNLF2b gene, and comprises amino acid residues 5-60 of the wild-type protein encoded by the BNLF2b gene.

In certain embodiments, the polypeptide comprises amino acid residues 5-61 of the wild-type protein encoded by the BNLF2b gene.

In certain embodiments, the polypeptide comprises amino acid residues 1-74 of the wild-type protein encoded by the BNLF2b gene.

In certain embodiments, the amino acid residues 1-74 have a sequence set forth in SEQ ID NO:89.

In certain embodiments, the polypeptide consists of at least 74 contiguous amino acid residues of the wild-type protein encoded by the BNLF2b gene.

In certain exemplary embodiments, the isolated polypeptide consists of a sequence selected from the group consisting of: amino acid residues 51-56, amino acid residues 1-25, amino acid residues 14-52, amino acid residues 1-52, amino acid residues 14-74, amino acid residues 11-65, amino acid residues 11-74, or amino acid residues 1-74 of the wild-type protein encoded by the BNLF2b gene. In certain exemplary embodiments, the isolated polypeptide consists of a sequence selected from the group consisting of SEQ ID NOs: 88-91, 97, 102-104.

In certain embodiments, the variant of the present invention differs from the polypeptide from which it is derived only by a substitution (e.g., conservative substitution or non-conservative substitution) of 1, 2, 3 or 4 amino acid residues, and retains a biological function of the polypeptide from which it is derived (e.g., an activity of being recognized and bound by an anti-EBV antibody).

In certain embodiments, the variant does not comprise an amino acid substitution at an amino acid position corresponding to a following position: 6, 9, 10, 11, 16, 31, 33, 38, 39, 53, 54, 56, 57, 58, 59, 95, 96, 97 with reference to amino acid positions set forth in the wild-type protein encoded by the BNLF2b gene.

In certain embodiments, the variant comprises an amino acid substitution at one or more (e.g., 1, 2, 3, or 4) amino acid positions corresponding to following positions: 5, 7, 8, 12, 13, 14, 15, 19, 22, 24, 25, 32, 34, 35, 36, 37, 40, 41, 42, 52, 55, 60, 61, 89, 91, 93 or 98 with reference to amino acid positions set forth in the wild-type protein encoded by the BNLF2b gene.

In certain embodiments, the variant comprises an amino acid substitution at one or more (e.g., 1, 2, 3, or 4) amino acid positions corresponding to following positions: 5, 7, 8, 12, 13, 14, 22, 25, 32, 34, 35, 36, 40, 41, 42, 52, 55, 60, 61, 89, 91, 93 or 98 with reference to amino acid positions set forth in the wild-type protein encoded by the BNLF2b gene.

In certain embodiments, the variant comprises one or several (e.g., 1, 2, 3, or 4) amino acid substitutions selected from the group consisting of: substitution of an amino acid at a position corresponding to position 5 with A, substitution of an amino acid at a position corresponding to position 7 with A, substitution of an amino acid at a position corresponding to position 8 with G, substitution of an amino acid at a position corresponding to position 12 with G, T, D or S, substitution of an amino acid at a position corresponding to position 13 with A, substitution of an amino acid at a position corresponding to position 14 with G, substitution of an amino acid at a position corresponding to position 15 with A, substitution of an amino acid at a position corresponding to position 22 with A, substitution of an amino acid at a position corresponding to position 24 with A, substitution of an amino acid at a position corresponding to position 25 with A, substitution of an amino acid at a position corresponding to position 32 with A, substitution of an amino acid at a position corresponding to position 34 with A, substitution of an amino acid at a position corresponding to position 35 with A, substitution of an amino acid at a position corresponding to position 36 with A, substitution of an amino acid at a position corresponding to position 37 with A, N, Q, S or R, substitution of an amino acid at a position corresponding to position 40 with A, substitution of an amino acid at a position corresponding to position 41 with A, substitution of an amino acid at a position corresponding to position 42 with A, substitution of an amino acid at a position corresponding to position 52 with K, H, A, S or D, substitution of an amino acid at a position corresponding to position 55 with S, substitution of an amino acid at a position corresponding to position 60 with A, substitution of an amino acid at a position corresponding to position 61 with K, H, S or A, substitution of an amino acid at a position corresponding to position 89 with A or T, substitution of an amino acid at a position corresponding to position 91 with A, substitution of an amino acid at a position corresponding to position 93 with Q, substitution of an amino acid at a position corresponding to position 98 with A.

In certain embodiments, the variant comprises one or several (e.g., 1, 2, 3, or 4) amino acid substitutions selected from the group consisting of: substitution of an amino acid at a position corresponding to position 5 with A, substitution of an amino acid at a position corresponding to position 7 with A, substitution of an amino acid at a position corresponding to position 8 with G, substitution of an amino acid at a position corresponding to position 12 with G, T, D or S, substitution of an amino acid at a position corresponding to position 13 with A, substitution of an amino acid at a position corresponding to position 14 with G, substitution of an amino acid at a position corresponding to position 22 with A, substitution of an amino acid at a position corresponding to position 25 with A, substitution of an amino acid at a position corresponding to position 32 with A, substitution of an amino acid at a position corresponding to position 34 with A, substitution of an amino acid at a position corresponding to position 35 with A, substitution of an amino acid at a position corresponding to position 36 with A, substitution of an amino acid at a position corresponding to position 40 with A, substitution of an amino acid at a position corresponding to position 41 with A, substitution of an amino acid at a position corresponding to position 42 with A, substitution of an amino acid at a position corresponding to position 52 with K, H, A, S or D, substitution of an amino acid at a position corresponding to position 55 with S, substitution of an amino acid at a position corresponding to position 60 with A, substitution of an amino acid at a position corresponding to position 61 with K, H, S or A, substitution of an amino acid at a position corresponding to position 89 with A or T, substitution of an amino acid at a position corresponding to position 91 with A, substitution of an amino acid at a position corresponding to position 93 with Q, substitution of an amino acid at a position corresponding to position 98 with A.

In certain embodiments, the isolated polypeptide consists of not more than 97 (e.g., not more than 96, not more than 95, not more than 94, not more than 93, not more than 92, not more than 91, not more than 90, not more than 89, not more than 88, not more than 87, not more than 86, not more than 85, not more than 84, not more than 83, not more than 82, not more than 81, not more than 80, not more than 79, not more than 78, not more than 76, not more than 75, or not more than 74) contiguous amino acid residues of the wild-type protein encoded by the BNLF2b gene.

In certain embodiments, the isolated polypeptide consists of 15-80 (e.g., 15-74) contiguous amino acid residues of the wild-type protein encoded by the BNLF2b gene. In certain embodiments, the isolated polypeptide consists of 39-74 contiguous amino acid residues of the wild-type protein encoded by the BNLF2b gene.

In one embodiment, the isolated polypeptide or variant thereof is attached to the surface of a solid support, or bears a modifying group capable of being attached to the solid support. In certain embodiments, the C-terminus of the isolated polypeptide or variant thereof is attached to the surface of the solid support, or bears a modifying group capable of being attached to the solid support. In certain embodiments, the modifying group is biotin or avidin. In certain embodiments, the solid support is selected from magnetic bead or microtiter plate (e.g., microwell plate or ELISA plate).

In another embodiment, the isolated polypeptide or variant thereof bears a detectable label. In certain embodiments, the detectable label is selected from the group consisting of enzyme (e.g., horseradish peroxidase or alkaline phosphatase), chemiluminescent reagent (e.g., acridine ester compound), fluorescent dye, or biotin.

### Preparation of polypeptide

The polypeptide or variant thereof of the present invention is not limited by its production method, for example, it can be produced by a genetic engineering method (recombinant technique) or chemical synthesis method.

In another aspect, the present invention provides an isolated nucleic acid molecule comprising a nucleotide sequence encoding the polypeptide or variant thereof of the present invention.

In another aspect, the present invention also provides a vector, which comprises the isolated nucleic acid molecule as described above. The vector of the present invention may be a cloning vector or an expression vector. In a preferred embodiment, the vector of the present invention is, for example, plasmid, cosmid, phage, cosmid or the like.

In another aspect, the present invention also provides a host cell comprising the isolated nucleic acid molecule or vector of the present invention. Such host cell includes, but is not limited to, prokaryotic cell such as *E. coli* cell, eukaryotic cell such as yeast cell, insect cell, plant cell, and animal cell (e.g., mammalian cell, such as mouse cell, human cell, etc.). The cell of the present invention may also be a cell line, such as 293T cell.

In another aspect, the present invention also provides a method for preparing the polypeptide or variant thereof of the present invention, which comprise, culturing the host cell of the present invention under conditions permitting the expression of the polypeptide or variant thereof, and recovering the polypeptide or variant thereof from a culture of the cultured host cell.

### Detection kit

In a second aspect, the present invention provides a kit, which comprises a capture reagent, and the capture reagent is selected from the isolated polypeptide or variant thereof of the first aspect. In certain embodiments, the kit further comprises an instruction for using the isolated polypeptide or variant thereof as a capture reagent to detect an antibody specific to BNLF2b gene-encoded protein in a sample, or an instruction for using the isolated polypeptide or variant thereof as a capture reagent to detecting the antibody level specific to BNLF2b gene-encoded protein in a sample from a subject, thereby determining whether the subject has nasopharyngeal cancer or is at risk for nasopharyngeal cancer.

In certain embodiments, the subject is a mammal, such as a human.

In some embodiments, the capture reagent is attached to a surface of a solid support. In certain embodiments, the C-terminus of the capture reagent is attached to a solid support. As used herein, the solid support includes concave well plate, test tube, bead (e.g., latex particle) or membrane (e.g., nitrocellulose membrane) which are made of or coated with a polymeric material such as polyvinyl chloride, polystyrene, polyacrylamide or cellulose, or magnetic bead pre-coated with a functional group (e.g., amino, carboxyl, biotin or avidin). In certain embodiments, the solid support is selected from magnetic bead or microtiter plate (e.g., microwell plate or ELISA plate).

In other embodiments, the capture reagent bears a modifying group capable of being attached to a solid support. In certain embodiments, the C-terminus of the capture reagent bears a modifying group capable of being attached to the solid support. In such embodiments, the kit may further comprise a coating reagent, such as coating buffer (e.g., carbonate buffer, phosphate buffer, Tris-HCL buffer, or borate buffer) for coating the capture reagent on the solid support. Methods for coating proteins or polypeptides on solid supports are well known in the art, and examples thereof include physical adsorption, covalent coupling via aminated or carboxylated surface, or binding mediated by avidin-biotin system, surface pre-coated with polylysine or surface pre-coated with protein A or protein G. In certain embodiments, the modifying group is biotin or avidin, and the surface of the solid support bears a corresponding linking group.

In certain embodiments, the kit comprises, in separate containers or in separate compartments of a single container unit, at least a solid support coated with avidin or streptavidin, and the above capture reagent.

In some embodiments, the method for determining whether a subject has nasopharyngeal cancer or is at risk for nasopharyngeal cancer by detecting anti-Epstein-Barr virus (EBV) antibody level in a sample from the subject is carried out by a double antigen sandwich method. Detection of the antibody level in a sample by double antigen sandwich method is well known to those skilled in the art. In such detection, "capture antigen" and "detection antigen" allow the antibody in sample to form a bridge between the two specific antigens, therefore the two antigens are usually the same, or have the same core epitope, or have immunological cross-reactivity, which allows one antibody to bind the two antigens.

Accordingly, in certain embodiments, the kit further comprises a detection reagent, and the detection reagent is selected from the isolated polypeptide or variant thereof of the first aspect.

In certain embodiments, the detection reagent bears a detectable label. In certain embodiments, the detectable label is selected from the group consisting of enzyme (e.g., horseradish peroxidase or alkaline phosphatase), chemiluminescent reagent (e.g., acridine ester compound), fluorescent dye, or biotin. In certain exemplary embodiments, the detectable label is selected from enzymes (e.g., horseradish peroxidase or alkaline phosphatase).

In certain embodiments, the detection reagent and the capture reagent comprise identical or substantially identical polypeptide sequence. In certain embodiments, the term "substantially identical" refers to that the two polypeptide sequences contained in the detection reagent and the capture reagent comprise the same core fragment, and the core fragment is selected from the group consisting of amino acid residues 5-11, amino acid residues 16-23, amino acid residues 31-39, and/or amino acid residues 53-60 (e.g., amino acid residues 53-61) of the wild-type protein encoded by the BNLF2b gene. In certain embodiments, the term "substantially identical" refers to that the two polypeptide sequences contained in the detection reagent and the capture reagent both comprise amino acid residues 53-60 (e.g., amino acid residues 53-61), amino acid residues 5-23, amino acid residues 16-39, amino acid residues 5-39, amino acid residues 16-60 (e.g., amino acid residues 16-61 amino acid residues), or amino acid residues 5-60 (e.g., amino acid residues 5-61) of the wild-type protein encoded by the BNLF2b gene. In certain embodiments, the term "substantially identical" refers to that the two polypeptide sequences contained in the detection reagent and the capture reagent both comprise amino acid residues 51-56, amino acid residues 1-25, amino acid residues 14-52, amino acid residues 1-52, amino acid residues 14-74, amino acid residues 11-65, amino acid residues 11-74, or amino acid residues 1-74 of the wild-type protein encoded by the BNLF2b gene.

In other embodiments, the method for determining whether a subject has nasopharyngeal cancer or is at risk for nasopharyngeal cancer by detecting a level of an anti-Epstein-Barr virus (EBV) antibody in a sample from the subject is performed by an indirect method. Detection of a level of an antibody in a sample by indirect methods is well known to those skilled in the art. In such detection, "capture antigen" first forms an immune complex with the antibody in the sample, and then the captured antibody is detected by a secondary antibody (e.g., an anti-immunoglobulin antibody).

Accordingly, in certain embodiments, the kit further comprises a detection reagent, and the detection reagent is selected from a secondary antibody bearing a detectable label. In certain embodiments, the detectable label is selected from the group consisting of enzyme (e.g., horseradish peroxidase or alkaline phosphatase), chemiluminescent reagent (e.g., acridine ester compound), fluorescent dye, or biotin. In certain exemplary embodiments, the detectable label is selected from enzymes (e.g., horseradish peroxidase or alkaline phosphatase).

In certain embodiments, the secondary antibody is specific to an antibody of a species (e.g., human) from which the antibody to be tested is derived.

In certain embodiments, the secondary antibody is an anti-immunoglobulin antibody.

In certain exemplary embodiments, the kit is used to detect an anti-EBV IgG antibody. In such embodiments, the anti-immunoglobulin antibody is selected from anti-IgG antibodies, such as anti-human IgG antibodies.

In certain exemplary embodiments, the kit is used to detect an anti-EBV IgM antibody. In such embodiments, the anti-immunoglobulin antibody is selected from anti-IgM antibodies, such as anti-human IgM antibodies.

In certain exemplary embodiments, the kit is used to detect an anti-EBV IgA antibody. In such embodiments, the anti-immunoglobulin antibody is selected from anti-IgA antibodies, such as anti-human IgA antibodies.

In certain embodiments, the kits of the present invention may further comprise one or more reagents or devices selected from the group consisting of: (i) a device for collecting or storing a sample from a subject (e.g., a blood collection device); (ii) an additional reagent (e.g., buffer, diluent, blocking solution, and/or standard) required to perform the detection.

### Detection use and method

In a third aspect, the present invention provides a method for detecting an antibody specific to BNLF2b gene-encoded protein in a sample, comprising the steps of:
(1) contacting the sample with a capture reagent to obtain an antigen-antibody immune complex; in which the capture reagent is selected from the isolated polypeptide or variant thereof described in the first aspect;
(2) determining an amount of the antigen-antibody immune complex obtained in step (1).

In certain embodiments, in step (2), the amount of the immune complex is determined by immunological assay.

In certain embodiments, the immunological assay is selected from enzyme immunoassay (e.g., ELISA), chemiluminescence immunoassay, fluorescent immunoassay, or radioimmunoassay.

In some embodiments, the capture reagent is attached to a surface of a solid support.

In other embodiments, the capture reagent bears a modifying group capable of being attached to a solid support. In such embodiments, prior to step (1), the method further comprises a step of coating the capture reagent on the surface of the solid support.

In certain embodiments, the C-terminus of the capture reagent is attached to the surface of the solid support, or has a modifying group capable of being attached to the solid support.

In some embodiments, the detection is performed by a double antigen sandwich method. Detection of a level of an antibody in a sample by double antigen sandwich method is well known to those skilled in the art. In such detection, "capture antigen" and "detection antigen" allow the sample antibody to form a bridge between the two specific antigens. Thus, the two antigens are usually the same, or have the same core epitope, or have immunological cross-reactivity, which allows one antibody to bind the two antigens.

Therefore, in certain embodiments, in step (2), the amount of the immune complex is detected by using a detection reagent selected from the isolated polypeptide or variant thereof described in the first aspect.

In certain embodiments, the detection reagent is selected from the isolated polypeptide or variant thereof of the first aspect, the isolated polypeptide or variant thereof bearing a detectable label. In certain embodiments, the detectable label is selected from the group consisting of enzyme (e.g., horseradish peroxidase or alkaline phosphatase), chemiluminescent reagent (e.g., acridine ester compound), fluorescent dye, or biotin. In certain exemplary embodiments, the detectable label is selected from enzymes (e.g., horseradish peroxidase or alkaline phosphatase).

In certain embodiments, the detection reagent and the capture reagent comprise identical or substantially identical polypeptide sequence. In certain embodiments, the term "substantially identical" refers to that the two polypeptide sequences contained in the detection reagent and the capture reagent comprise the same core fragment, and the core fragment is selected from the group consisting of amino acid residues 5-11, amino acid residues 16-23, amino acid residues 31-39, and/or amino acid residues 53-60 (e.g., amino acid residues 53-61) of the wild-type protein encoded by the BNLF2b gene. In certain embodiments, the term "substantially identical" refers to that the two polypeptide sequences contained in the detection reagent and the capture reagent both comprise amino acid residues 53-60 (e.g., amino acid residues 53-61), amino acid residues 5-23, amino acid residues 16-39, amino acid residues 5-39, amino acid residues 16-60 (e.g., amino acid residues 16-61 amino acid residues), or amino acid residues 5-60 (e.g., amino acid residues 5-61) of the wild-type protein encoded by the BNLF2b gene. In certain embodiments, the term "substantially identical" refers to that the two polypeptide sequences contained in the detection reagent and the capture reagent both comprise amino acid residues 51-56, amino acid residues 1-25, amino acid residues 14-52, amino acid residues 1-52, amino acid residues 14-74, amino acid residues 11-65, amino acid residues 11-74, or amino acid residues 1-74 of the wild-type protein encoded by the BNLF2b gene.

In other embodiments, the detection is performed by an indirect method. Detection of a level of an antibody in a sample by indirect methods is well known to those skilled in the art. In such detection, the "capture antigen" first forms an immune complex with the antibody in the sample, and then the captured antibody is detected by a secondary antibody (e.g., an anti-immunoglobulin antibody).

Thus, in certain embodiments, in step (2), an amount of the immune complex is detected using a detection reagent selected from a secondary antibody bearing a detectable label.

In certain embodiments, the detectable label is selected from the group consisting of enzyme (e.g., horseradish peroxidase or alkaline phosphatase), chemiluminescent reagent (e.g., acridine ester compound), fluorescent dye, or biotin.

In certain embodiments, the secondary antibody is specific to an antibody of a species (e.g., human) from which the antibody to be tested is derived.

In certain embodiments, the secondary antibody is an anti-immunoglobulin antibody.

In certain exemplary embodiments, the antibody to be detected is an IgG antibody. In such embodiments, the anti-immunoglobulin antibody is selected from anti-IgG antibodies, such as anti-human IgG antibodies.

In certain exemplary embodiments, the antibody to be detected is an IgM antibody. In such embodiments, the anti-immunoglobulin antibody is selected from anti-IgM antibodies, such as anti-human IgM antibodies.

In certain exemplary embodiments, the antibody to be detected is an IgA antibody. In such embodiments, the anti-immunoglobulin antibody is selected from anti-IgA antibodies, such as anti-human IgA antibodies.

In another aspect, the present invention also relates to a use of the isolated polypeptide or variant thereof of the first aspect in the manufacture of a kit for detecting an antibody specific to BNLF2b gene-encoded protein in a sample. In certain embodiments, the kit detects the antibody specific to BNLF2b gene-encoded protein in the sample by the method described in the third aspect.

### Diagnostic use and method

In a fourth aspect, the present invention provides a method for determining whether a subject has nasopharyngeal cancer or is at risk for nasopharyngeal cancer, comprising:
(1) determining a level of an antibody specific to BNLF2b gene-encoded protein in a sample from the subject; and,
(2) comparing the level with a reference value.

In certain embodiments, the subject is considered to have or be at risk for nasopharyngeal cancer if the level is above the reference value.

Herein, the reference value is a value derived from a subject without nasopharyngeal cancer or a healthy person (e.g., a subject with no detectable disease and no history of cancer or nasopharyngeal cancer), or a level of an antibody specific to BNLF2b gene-encoded protein in a corresponding sample from a subject without nasopharyngeal cancer or a healthy person.

In certain embodiments, the sample is a blood sample, such as whole blood, plasma, or serum.

In certain embodiments, the subject is a mammal, such as a human.

In certain embodiments, the level of the antibody specific to BNLF2b gene-encoded protein in the sample is determined by an immunological assay. In certain embodiments, the immunological assay is selected from the group consisting of enzyme immunoassay (e.g., ELISA), chemiluminescence immunoassay, fluorescent immunoassay, or radioimmunoassay.

In certain embodiments, the detection comprises using the isolated polypeptide or variant thereof of the first aspect as a capture reagent.

In certain embodiments, step (1) comprises the steps of:
(1a) contacting a sample from the subject with a capture reagent to obtain an antigen-antibody immune complex; in which the capture reagent is selected from the isolated polypeptide or variant thereof described in the first aspect;
(1b) determining an amount of the antigen-antibody immune complex obtained in step (1a).

In certain embodiments, in step (1b), the amount of the immune complex is determined by immunological assay. In certain embodiments, the immunological assay is selected from enzyme immunoassay (e.g., ELISA), chemiluminescence immunoassay, fluorescent immunoassay, or radioimmunoassay.

In some embodiments, the capture reagent is attached to a surface of a solid support.

In other embodiments, the capture reagent bears a modifying group capable of being attached to a solid support. In such embodiments, prior to step (1), the method further comprises the step of coating the capture reagent on the surface of the solid support.

In certain embodiments, the C-terminus of the capture reagent is attached to the surface of the solid support, or has a modifying group capable of being attached to the solid support.

In some embodiments, the detection is performed by a double antigen sandwich method. Detection of a level of an antibody in a sample by double antigen sandwich method is well known to those skilled in the art. In such determination, "capture antigen" and "detection antigen" allow the antibody in sample to form a bridge between the two specific antigens, therefore the two antigens are usually the same, or have the same core epitope, or having immunological cross-reactivity, which allows one antibody to bind the two antigens.

Therefore, in certain embodiments, in step (1b), the amount of the immune complex is detected using a detection reagent, the detection reagent is selected from the isolated polypeptide or variant thereof described in the first aspect.

In certain embodiments, the detection reagent is selected from the isolated polypeptide or variant thereof of the first aspect, and the isolated polypeptide or variant thereof bears a detectable label. In certain embodiments, the detectable label is selected from the group consisting of enzyme (e.g., horseradish peroxidase or alkaline phosphatase), chemiluminescent reagent (e.g., acridine ester compound), fluorescent dye, or biotin. In certain exemplary embodiments, the detectable label is selected from enzymes (e.g., horseradish peroxidase or alkaline phosphatase).

In certain embodiments, the detection reagent and the capture reagent comprise identical or substantially identical polypeptide sequence. In certain embodiments, the term "substantially identical" refers to that the two polypeptide sequences contained in the detection reagent and the capture reagent comprise the same core fragment, and the core fragment is selected from the group consisting of amino acid residues 5-11, amino acid residues 16-23, amino acid residues 31-39, and/or amino acid residues 53-60 (e.g., amino acid residues 53-61) of the wild-type protein encoded by the BNLF2b gene. In certain embodiments, the term "substantially identical" refers to that the two polypeptide sequences contained in the detection reagent and the capture reagent both comprise amino acid residues 53-60 (e.g., amino acid residues 53-61), amino acid residues 5-23, amino acid residues 16-39, amino acid residues 5-39, amino acid residues 16-60 (e.g., amino acid residues 16-61 amino acid residues), or amino acid residues 5-60 (e.g., amino acid residues 5-61) of the wild-type protein encoded by the BNLF2b gene. In certain embodiments, the term "substantially identical" refers to that the two polypeptide sequences contained in the detection reagent and the capture reagent both comprise amino acid residues 51-56, amino acid residues 1-25, amino acid residues 14-52, amino acid residues 1-52, amino acid residues 14-74, amino acid residues 11-65, amino acid residues 11-74, or amino acid residues 1-74 of the wild-type protein encoded by the BNLF2b gene.

In other embodiments, the detection is performed by an indirect method. Detection of a level of an antibody in a sample by indirect methods is well known to those skilled in the art. In such detection, "capture antigen" first forms an immune complex with the antibody in the sample, and then the captured antibody is detected by a secondary antibody (e.g., an anti-immunoglobulin antibody).

Thus, in certain embodiments, in step (1b), an amount of the immune complex is detected using a detection reagent, and the detection reagent is selected from a secondary antibody bearing a detectable label.

In certain embodiments, the detectable label is selected from the group consisting of enzyme (e.g., horseradish peroxidase or alkaline phosphatase), chemiluminescent reagent (e.g., acridine ester compound), fluorescent dye, or biotin.

In certain embodiments, the secondary antibody is specific to an antibody of a species (e.g., human) from which the antibody to be tested is derived.

In certain embodiments, the secondary antibody is an anti-immunoglobulin antibody.

In certain exemplary embodiments, the antibody to be detected is an IgG antibody. In such embodiments, the anti-immunoglobulin antibody is selected from anti-IgG antibodies, such as anti-human IgG antibodies.

In certain exemplary embodiments, the antibody to be detected is an IgM antibody. In such embodiments, the anti-immunoglobulin antibody is selected from anti-IgM antibodies, such as anti-human IgM antibodies.

In certain exemplary embodiments, the antibody to be detected is an IgA antibody. In such embodiments, the anti-immunoglobulin antibody is selected from anti-IgA antibodies, such as anti-human IgA antibodies.

In certain embodiments, the method further comprises: prior to step (1), providing a sample from the subject.

In certain embodiments, the method further comprises: after step (2), administering to a subject considered to have or be at risk for nasopharyngeal cancer a therapeutically effective amount of an anti-tumor therapy (e.g., chemotherapy, radiation therapy, and/or immunotherapy) capable of treating nasopharyngeal cancer.

In certain embodiments, the anti-tumor therapy capable of treating nasopharyngeal cancer is selected from the group consisting of surgery, radiation therapy (e.g., external radiation therapy EBRT, brachytherapy), or chemotherapy (e.g., carboplatin, paclitaxel, docetaxel, gemcitabine, doxorubicin, epirubicin, bleomycin, methotrexate), targeted therapy (e.g., cetuximab), immunotherapy (e.g., PD-1 mAb), and combination of various therapies (e.g., radiotherapy + chemotherapy, radiotherapy + surgery).

In another aspect, the present invention also relates to a use of a reagent capable of detecting the antibody level specific to BNLF2b gene-encoded protein in the manufacture of a kit, and the kit is used for determining whether a subject has nasopharyngeal cancer or is at risk for nasopharyngeal cancer.

In certain embodiments, the reagent is capable of detecting the antibody level specific to BNLF2b gene-encoded protein by an immunological assay. In certain embodiments, the immunological assay is selected from the group consisting of enzyme immunoassay (e.g., ELISA), chemiluminescence immunoassay, fluorescent immunoassay, or radioimmunoassay.

In certain embodiments, the subject is a mammal, such as a human.

In certain embodiments, the sample is a blood sample, such as whole blood, plasma, or serum.

In certain embodiments, the kit determines whether a subject has nasopharyngeal cancer or is at risk for nasopharyngeal cancer by the following method:
(1) detecting the antibody level specific to BNLF2b gene-encoded protein in a sample from the subject; and,
(2) comparing the level with a reference value.

In certain embodiments, if the level is higher than the reference value, the subject is considered to have nasopharyngeal cancer or be at risk for nasopharyngeal cancer.

In certain embodiments, the reagent capable of detecting the antibody level specific to BNLF2b gene-encoded protein comprises a capture reagent, and the capture reagent is selected from the isolated polypeptide or variant thereof of the first aspect.

In certain embodiments, the above-mentioned step (1) comprises the following steps:
(1a) contacting a sample from the subject with a capture reagent to obtain an antigen-antibody immune complex; in which the capture reagent is selected from the isolated polypeptide or variant thereof described in the first aspect;
(1b) determining an amount of the antigen-antibody immune complex obtained in step (1a).

In certain embodiments, in step (1b), the amount of the immune complex is determined by immunological assay. In certain embodiments, the immunological assay is selected from enzyme immunoassay (e.g., ELISA), chemiluminescence immunoassay, fluorescent immunoassay, or radioimmunoassay.

In some embodiments, the capture reagent is attached to a surface of a solid support.

In other embodiments, the capture reagent bears a modifying group capable of being attached to a solid support. In such embodiments, prior to step (1) above, the method further comprises a step of coating the capture reagent on the surface of the solid support.

In certain embodiments, the C-terminus of the capture reagent is attached to the surface of the solid support, or has a modifying group that can be attached to the solid support.

In some embodiments, the reagent capable of determining a level of an antibody specific to BNLF2b gene-encoded protein further comprises a detection reagent, and the detection reagent is selected from the isolated polypeptide or variant thereof described in the first aspect.

In certain embodiments, in the above step (1b), the detection reagent is used to detect the amount of the immune complex.

In certain embodiments, the detection reagent is selected from the isolated polypeptide or variant thereof of the first aspect, and the isolated polypeptide or variant thereof bears a detectable label. In certain embodiments, the detectable label is selected from the group consisting of enzyme (e.g., horseradish peroxidase or alkaline phosphatase), chemiluminescent reagent (e.g., acridine ester compound), fluorescent dye, or biotin. In certain exemplary embodiments, the detectable label is selected from enzymes (e.g., horseradish peroxidase or alkaline phosphatase).

In certain embodiments, the detection reagent and the capture reagent comprise identical or substantially identical polypeptide sequence. In certain embodiments, the term "substantially identical" refers to that the two polypeptide sequences contained in the detection reagent and the capture reagent comprise the same core fragment, and the core fragment is selected from the group consisting of amino acid residues 5-11, amino acid residues 16-23, amino acid residues 31-39, and/or amino acid residues 53-60 (e.g., amino acid residues 53-61) of the wild-type protein encoded by the BNLF2b gene. In certain embodiments, the term "substantially identical" refers to that the two polypeptide sequences contained in the detection reagent and the capture reagent both comprise amino acid residues 53-60 (e.g., amino acid residues 53-61), amino acid residues 5-23, amino acid residues 16-39, amino acid residues 5-39, amino acid residues 16-60 (e.g., amino acid residues 16-61 amino acid residues), or amino acid residues 5-60 (e.g., amino acid residues 5-61) of the wild-type protein encoded by the BNLF2b gene. In certain embodiments, the term "substantially identical" refers to that the two polypeptide sequences contained in the detection reagent and the capture reagent both comprise amino acid residues 51-56, amino acid residues 1-25, amino acid residues 14-52, amino acid residues 1-52, amino acid residues 14-74, amino acid residues 11-65, amino acid residues 11-74, or amino acid residues 1-74 of the wild-type protein encoded by the BNLF2b gene.

In other embodiments, the reagent capable of detecting the antibody level specific to BNLF2b gene-encoded protein further comprises a detection reagent, and the detection reagent is selected from a secondary antibody bearing a detectable label.

In certain embodiments, in the above step (1b), the detection reagent is used to detect the amount of the immune complex.

In certain embodiments, the detectable label is selected from the group consisting of enzyme (e.g., horseradish peroxidase or alkaline phosphatase), chemiluminescent reagent (e.g., acridine ester compound), fluorescent dye, or biotin.

In certain embodiments, the secondary antibody is specific to an antibody of a species (e.g., human) from which the antibody to be tested is derived.

In certain embodiments, the secondary antibody is an anti-immunoglobulin antibody.

In certain exemplary embodiments, the antibody to be detected is an IgG antibody. In such embodiments, the anti-immunoglobulin antibody is selected from anti-IgG antibodies, such as anti-human IgG antibodies.

In certain exemplary embodiments, the antibody to be detected is an IgM antibody. In such embodiments, the anti-immunoglobulin antibody is selected from anti-IgM antibodies, such as anti-human IgM antibodies.

In certain exemplary embodiments, the antibody to be detected is an IgA antibody. In such embodiments, the anti-immunoglobulin antibody is selected from anti-IgA antibodies, such as anti-human IgA antibodies.

### Definition of terms

In the present invention, unless otherwise specified, the scientific and technical terms used herein have the meanings commonly understood by those skilled in the art. Moreover, the laboratory procedures of virology, biochemistry, and immunology used herein are all routine procedures widely used in the corresponding fields. Meanwhile, for a better understanding of the present invention, definitions and explanations of related terms are provided below.

As used herein, the term "BNLF2b" refers to the BNLF2b gene of Epstein-Barr virus (EBV), which is well known to those skilled in the art (see, for example, NCBI GENBANK database accession number: CAA24811.1). The full-length protein encoded by the BNLF2b gene contains 98 amino acids, and its sequence is set forth in SEQ ID NO:101. Herein, the full-length protein encoded by the BNLF2b gene may also be referred to as the wild-type protein encoded by the BNLF2b gene, or BNLF2b gene-encoded protein.

As used herein, when referring to an amino acid sequence of BNLF2b gene-encoded protein, it is described by reference to the sequence set forth in SEQ ID NO:101. For example, the expression "amino acid residues 53-60 of the wild-type protein encoded by the BNLF2b gene" refers to amino acid residues 53-60 of the polypeptide set forth in SEQ ID NO: 101. However, those skilled in the art understand that, in the amino acid sequence of the protein encoded by the BNLF2b gene, mutations or variations can be naturally generated or artificially introduced without affecting its biological function. Therefore, in the present invention, the term " BNLF2b gene-encoded protein" and similar expressions shall include all such sequences, including, for example, the sequence set forth in SEQ ID NO: 101 and natural or artificial variants thereof. And, when describing a sequence fragment of BNLF2b gene-encoded protein, it includes not only a sequence fragment of SEQ ID NO: 101, but also a corresponding sequence fragment of its natural or artificial variants. For example, the expression "amino acid residues 53-60 of BNLF2b gene-encoded protein" includes amino acid residues 53-60 of SEQ ID NO: 101, and corresponding fragments in its (natural or artificial) variants. According to the present invention, the expression "corresponding sequence fragment" or "corresponding fragment" refers to a fragment located in equivalent position of the sequences being compared when the sequences are optimally aligned, i.e., when the sequences are aligned to obtain the highest percent identity.

As used herein, the term "specific binding" refers to a non-random binding reaction between two molecules (i.e., a binding molecule and a target molecule), such as a reaction between an antibody and an antigen to which it is directed. The binding affinity between two molecules can be described by K_{D} value. The K_{D} value is a dissociation constant derived from the ratio of K_{D} (dissociation rate of a specific binding molecule-target molecule interaction; also known as koff) to ka (association rate of a specific binding molecule-target molecule interaction; also known as kon), or refers to kd/ka expressed as molar concentration (M). The smaller the K_{D} value, the tighter the binding between the two molecules and the higher the affinity. In certain embodiments, an antibody that specifically binds to an antigen (or an antibody specific to an antigen) refers to that the antibody binds to the antigen with an affinity (K_{D}) of less than about 10⁻⁵ M, for example less than about 10⁻⁶ M, 10⁻⁷ M, 10⁻⁸ M, 10⁻⁹ M or 10⁻¹⁰ M or less. K_{D} values can be determined by methods well known in the art, such as by using surface plasmon resonance (SPR) in a BIACORE instrument.

As used herein, the term "immunological assay" refers to an assay that utilizes the specific interaction/binding affinity between antigen-antibody, which can generally be used to detect the presence or level of a specific antigen or antibody in a sample. Such immunological assays are well known to those skilled in the art and include, but are not limited to, enzyme immunoassay (EIA), chemiluminescence immunoassay (CLIA), radioimmunoassay (RIA), fluorescence immunoassay (FIA), Western blotting, immunoturbidimetric method, surface plasmon resonance method, etc. In certain embodiments, the immunological assay is enzyme immunoassay (EIA), such as ELISA assay, Elispot assay, or CLEIA assay. For a detailed description of immunological assays, see, for example, Fundamental Immunology, Ch. 7 Paul, W., ed., 2nd ed., Raven Press, N.Y. (1989).

As used herein, the term "epitope" refers to a site on an antigen that is specifically bound by an immunoglobulin or antibody. "Epitope" is also known in the art as "antigenic determinant". For example, epitope typically includes at least 3, 4, 5, 6, 7, 8, 9, 10, 11, 12, 13, 14 or 15 contiguous or non-contiguous amino acids in a unique spatial conformation, which may be "linear" or "conformational". In a linear epitope, all the interaction sites between a protein and an interacting molecule (e.g., antibody) exist linearly along the primary amino acid sequence of the protein. In a conformational epitope, the interaction sites exist across amino acid residues that are separated from each other in the protein.

As used herein, the term "detectable label" can be any substance detectable by fluorescent, spectroscopic, photochemical, biochemical, immunological, electrical, optical or chemical means. It is particularly preferred that such label is suitable for use in immunological detection (e.g., enzyme-linked immunoassay, radioimmunoassay, fluorescent immunoassay, chemiluminescence immunoassay, etc.). Such label is well known in the art and includes, but is not limited to, enzyme (e.g., horseradish peroxidase, alkaline phosphatase, β-galactosidase, urease, glucose oxidase, etc.), radionuclide (e.g., ³H, ¹²⁵I, ³⁵S, ¹⁴C, or ³²P), fluorescent dye (e.g., fluorescein isothiocyanate (FITC), fluorescein, tetramethylrhodamine isothiocyanate (TRITC), phycoerythrin (PE), Texas red, rhodamine, quantum dot or cyanine derivative (e.g., Cy7, Alexa 750), luminescent substance (e.g., chemiluminescent substance, such as acridine ester compound), magnetic bead (e.g., Dynabeads^{®} ), calorimetric label such as colloidal gold or colored glass or plastic (e.g., polystyrene, polypropylene, latex, etc.) beads, and biotin for binding to avidin (e.g., streptavidin) modified with the aforementioned label. Patents teaching the use of such label include, but are not limited to, US Patents 3,817,837; 3,850,752; 3,939,350; 3,996,345; 4,277,437; 4,275,149; and 4,366,241 (all of them are incorporated herein by reference). Labels encompassed by the present invention can be detected by methods known in the art. For example, the radiolabel can be detected using photographic film or a scintillation calculator, and the fluorescent label can be detected using a light detector to detect the emitted light. The enzyme label is generally detected by providing a substrate to the enzyme and detecting the reaction product produced by the action of the enzyme on the substrate, and the calorimetric label is detected by simply visualizing the colored label. In certain embodiments, a detectable label as described above can be attached to the detection antibody or antigen via a linker of different lengths to reduce potential steric hindrance.

As used herein, the term "antibody" refers to an immunoglobulin molecule generally composed of two pairs of polypeptide chains (each pair having one light chain (LC) and one heavy chain (HC)). Antibody light chains can be classified as κ (kappa) and λ (lambda) light chains. Heavy chains can be classified as µ, δ, γ, α, or ε, and accordingly the isotypes of antibody are defined as IgM, IgD, IgG, IgA and IgE, respectively. Within the light and heavy chains, variable and constant regions are linked by a "J" region of about 12 or more amino acids, and the heavy chain also contains a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. Constant domains are not directly involved in the binding of antibody to antigen, but exhibit a variety of effector functions, such as mediating the binding of immunoglobulin with host tissue or factor, including various cells of the immune system (e.g., effector cells) and classical complement system first component (C1q). VH and VL regions can also be subdivided into regions of high variability (called complementarity determining regions (CDRs) interspersed with more conserved regions called framework regions (FRs). Each V_{H} and V_{L} consists of 3 CDRs and 4 FRs arranged in the following order: FR1, CDR1, FR2, CDR2, FR3, CDR3, FR4 from the amino terminus to the carboxy terminus. The variable regions (VH and VL) of each heavy chain/light chain pair form the antigen binding site.

As used herein, the term "isolated" or "being isolated" refers to artificially obtained from the natural state. If an "isolated" substance or component occurs in nature, it may be due to a change in its natural environment, or separation of the substance from its natural environment, or both. For example, a certain unisolated polynucleotide or polypeptide naturally exists in a living animal, and the same polynucleotide or polypeptide with high purity isolated from this natural state is called "being isolated". The terms "isolated" or "being isolated" do not exclude the admixture of artificial or synthetic substances, nor the presence of other impure substances that do not affect the activity of the substance.

As used herein, the term "vector" refers to a nucleic acid delivery vehicle into which a polynucleotide can be inserted. When the vector enables the expression of a protein encoded by the inserted polynucleotide, the vector is called an expression vector. The vector can be introduced into a host cell by transformation, transduction or transfection, so that the genetic material elements carried by it can be expressed in the host cell. Vectors are well known to those skilled in the art and include, but are not limited to: plasmid; phagemid; cosmid; artificial chromosome such as yeast artificial chromosome (YAC), bacterial artificial chromosome (BAC) or P1 derived artificial chromosome (PAC); phage such as λ phage or M13 phage and animal virus. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papillomavirus, papovavirus (e.g., SV40). A vector may contain a variety of elements that control expression, including, but not limited to, promoter sequence, transcription initiation sequence, enhancer sequence, selection element, and reporter gene. Additionally, the vector may also contain an origin of replication.

As used herein, the term "host cell" refers to a cell into which a vector can be introduced, which includes, but not is limited to, prokaryotic cell such as *E. coli* or *Bacillus subtilis,* fungal cell such as yeast cell or *Aspergillus,* etc., insect cell such as *S2 Drosophila* cell or Sf9, or animal cell such as fibroblast, CHO cell, COS cell, NSO cell, HeLa cell, BHK cell, HEK 293 cell or human cell.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison × 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

As used herein, the term "conservative substitution" refers to amino acid substitutions which would not disadvantageously affect or change the expected properties of a protein/polypeptide comprising the amino acid sequence. For example, a conservative substitution may be introduced by standard techniques known in the art such as site-directed mutagenesis and PCR-mediated mutagenesis. Conservative amino acid substitutions include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having basic side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, tryptophan), amino acids having nonpolar side chains (for example, alanine, valine, leucine, isoleucine, proline, phenylalanine, methionine), amino acids having β-branched side chains (such as threonine, valine, isoleucine) and amino acids having aromatic side chains (for example, tyrosine, phenylalanine, tryptophan, histidine). Therefore, a corresponding amino acid residue is preferably substituted with another amino acid residue from the same side-chain family. Methods for identifying amino acid conservative substitutions are well known in the art (see, for example, Brummell et al., Biochem. 32: 1180-1187 (1993); Kobayashi et al., Protein Eng. 12(10): 879-884 (1999); and Burks et al., Proc. Natl Acad. Set USA 94: 412-417 (1997), which are incorporated herein by reference).

The twenty conventional amino acids covered herein are written to follow conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

As used herein, the term "subject" includes, but is not limited to, various animals, particularly mammals such as human.

### Beneficial effect

The present invention discovers for the first time that the level of antibody against protein encoded by EBV BNLF2b gene can be used to diagnose nasopharyngeal cancer or assess the risk for nasopharyngeal cancer, and its diagnostic performance is significantly better than the existing markers, which can significantly improve the specificity and positive predictive value for nasopharyngeal cancer screening. The present invention also provides for the first time a serological screening kit for nasopharyngeal cancer based on a protein or polypeptide fragment encoded by EBV BNLF2b gene.

Compared with the prior art, the technical solution of the present invention can achieve a detection sensitivity comparable to that of the known combined detection of EBV antibodies, and an even better detection specificity, and can realize rapid and high-throughput detection, thereby having significant clinical value.

The embodiments of the present invention will be described in detail below with reference to the drawings and examples, but those skilled in the art will understand that the following drawings and examples are only used to illustrate the present invention, rather than limit the scope of the present invention. Various objects and advantageous aspects of the present invention will become apparent to those skilled in the art from the accompanying drawings and the following detailed description of the preferred embodiments.

### Brief Description of the Drawings

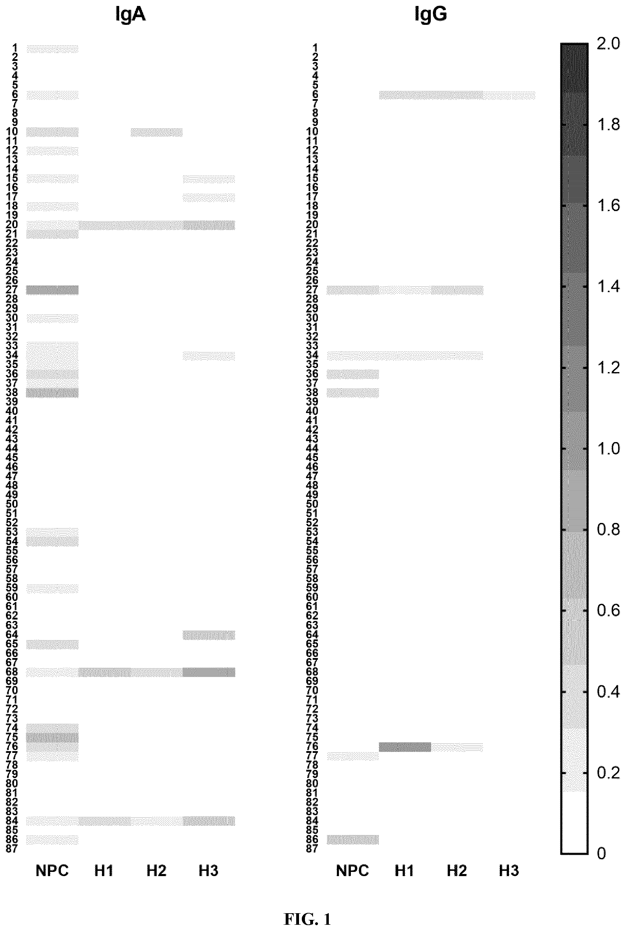

FIG. 1 shows the reactivity of IgA and IgG antibodies in one pooled serum sample of nasopharyngeal cancer (NPC) and three serum samples of healthy control (H1 to H3) with 87 EBV polypeptides (numbers 1-87) in Examples 2 and 3. The left diagram represents IgA antibody, the right diagram represents IgG antibody, the abscissa represents different serum samples, and the ordinate represents different EBV polypeptides, in which the darker the color, the higher the reactivity.

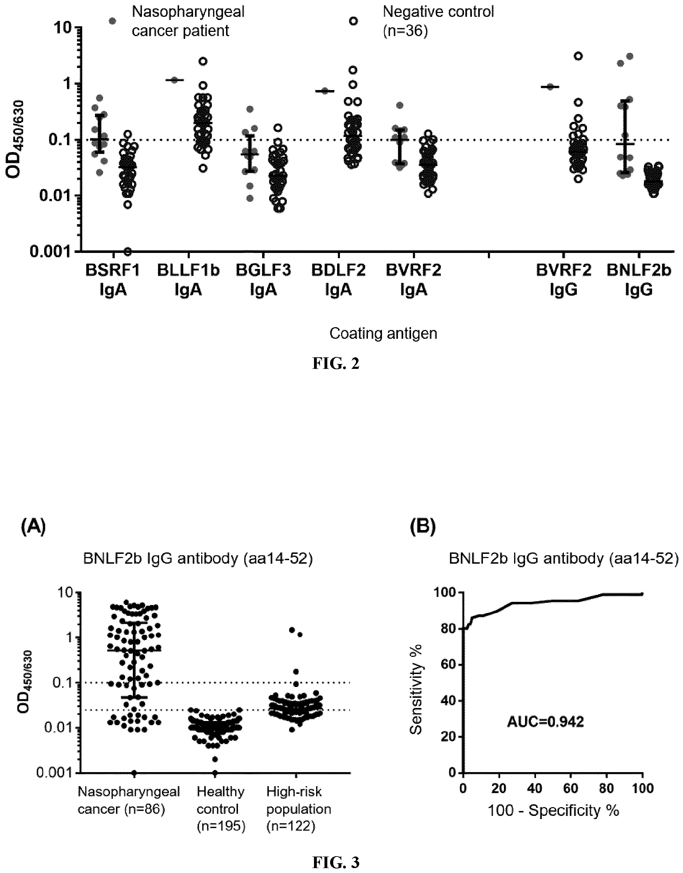

FIG. 2 shows the reactivity of antibodies in serum samples from nasopharyngeal cancer patients and healthy controls with different EBV polypeptides in Examples 2 and 3, in which the abscissa represents EBV polypeptide, and the ordinate represents reaction OD value (logarithmic scale).
FIG. 3 shows the reactivity of IgG antibodies in serum samples of nasopharyngeal cancer patients and healthy controls with BNLF2b-encoded protein aa14-52 in Example 4. FIG. 3A shows the detected OD values (logarithmic scale) of different samples; FIG. 3B shows the results of ROC curve analysis, in which the abscissa represents 100-specificity %, and the ordinate represents sensitivity %.

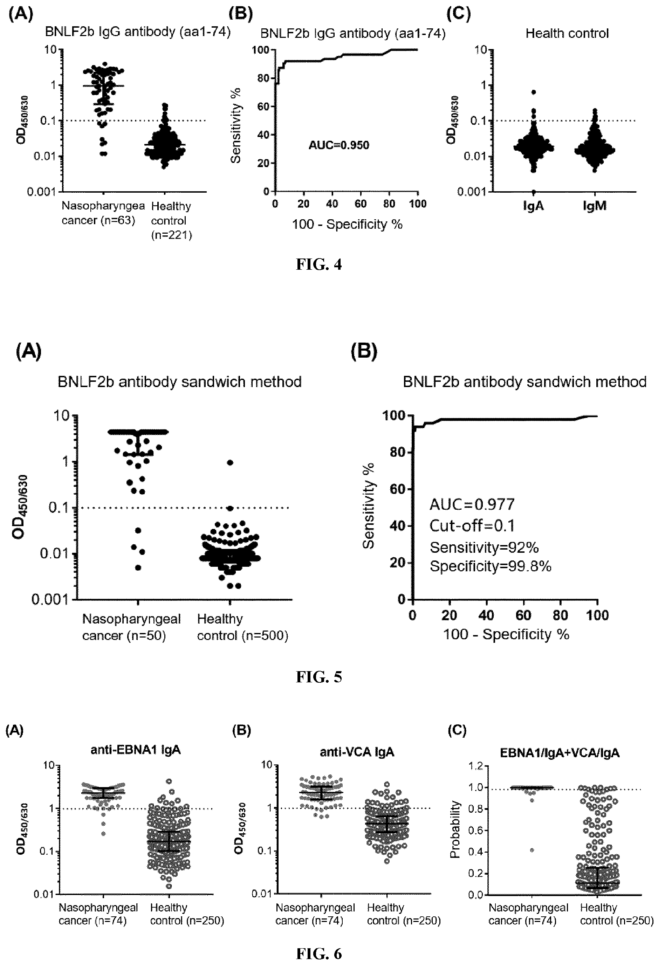

FIG. 4 shows the reactivity of IgA, IgM and IgG antibodies in serum samples of nasopharyngeal cancer patients and healthy controls with BNLF2b-encoded protein aa1-74 in Example 4. FIG. 4A shows the detected OD values, the abscissa represents serum classification, and the ordinate represents OD value (logarithmic scale); FIG. 4B shows the results of ROC curve analysis, the abscissa represents 100-specificity%, and the ordinate represents sensitivity%; FIG. 4C shows the reactivity of IgA and IgM antibodies in serum sample of healthy control with BNLF2b-encoded protein aa1-74, the abscissa represents antibody type, and the ordinate represents OD value (logarithmic scale).
FIG. 5 shows the detection results of 50 serum samples of nasopharyngeal cancer and 500 serum samples of healthy control by the double-antigen sandwich method for BNLF2b antibody in Example 5. FIG. 5A shows the detection OD values, the abscissa represents serum classification, and the ordinate represents OD value (logarithmic scale); FIG. 5B shows the results of ROC curve analysis, the abscissa represents 100-specificity%, and the ordinate represents sensitivity%.
FIG. 6 shows the EBNA1/IgA detection and VCA/IgA detection of 74 serum samples of nasopharyngeal cancer and 250 serum samples of healthy control, and the probability of nasopharyngeal cancer calculated by the combined detection of the two in Example 6. The abscissa represents serum classification, the ordinates in FIG. 6A and FIG. 6B represent detected OD value (logarithmic scale), and the ordinate in FIG. 6C represents the probability of nasopharyngeal cancer.

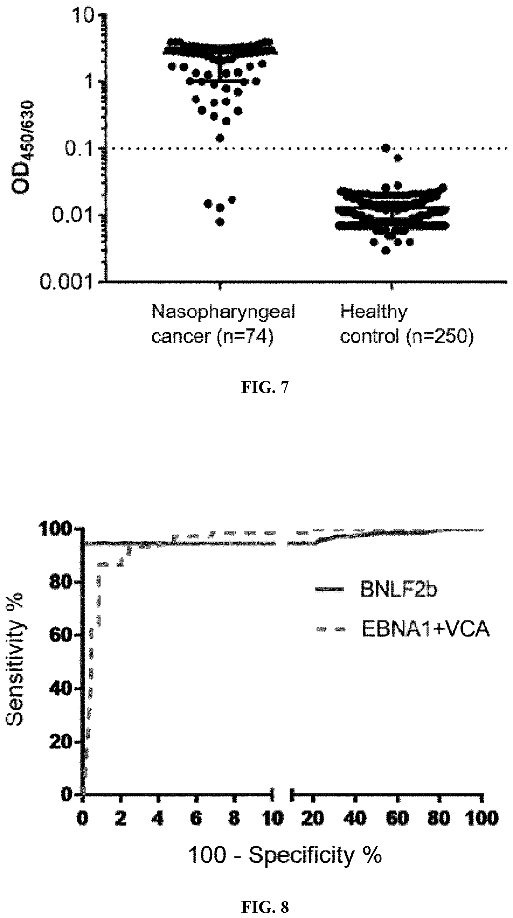

FIG. 7 shows the detection results of serum samples of nasopharyngeal cancer patients and healthy controls detected by the double-antigen sandwich method for BNLF2b antibody in Example 6, in which the abscissa represents serum classification, and the ordinate represents reaction OD value.
FIG. 8 shows the ROC curves of the double-antigen sandwich method for BNLF2b antibody and the combined detection of EBNA1/IgA+VCA/IgA in Example 6, in which the abscissa represents 100-specificity %, and the ordinate represents sensitivity %.

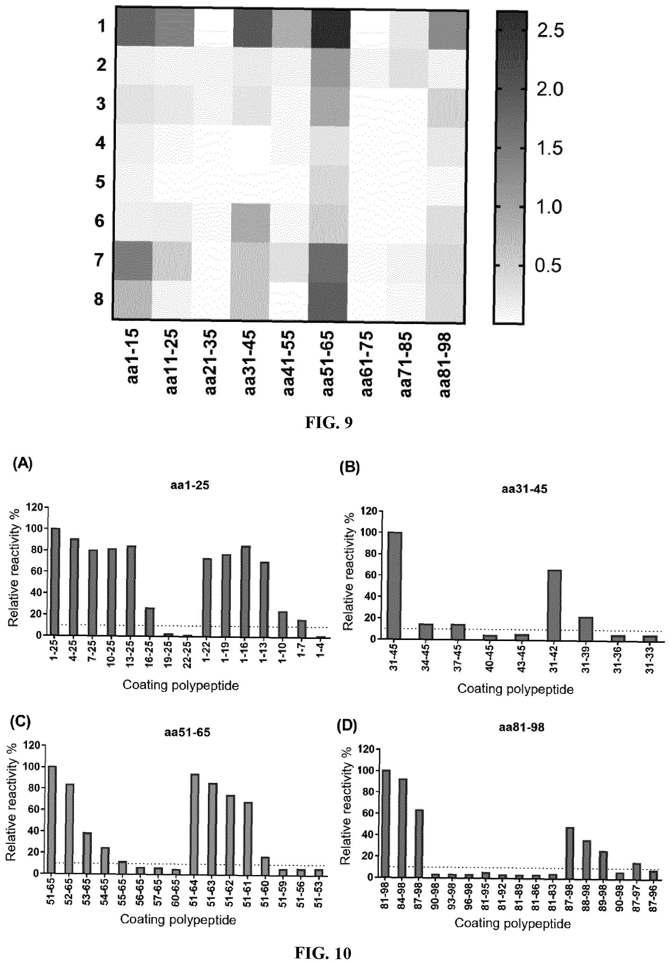

FIG. 9 shows the reactivity of IgG antibody in serum sample of nasopharyngeal cancer with different peptide fragments of the BNLF2b-encoded protein in Example 10, in which the abscissa represents different BNLF2b polypeptides, and the ordinate represents the serial number of pooled serum sample of nasopharyngeal cancer, in which the darker the color, the higher the reactivity.
FIG. 10 shows the reactivity of IgG antibody in two pooled serum samples of nasopharyngeal cancer with different peptide segments of the BNLF2b-encoded protein in Example 10, in which A to D represent the reactivity of the serum samples of nasopharyngeal cancer with the truncated peptides in the aa1-25 segment, the truncated peptides in the aa31-45 segment, the truncated peptides in the aa51-65 segment and the truncated peptides in the aa81-98 segment, respectively; the abscissa represents polypeptide position (e.g., 1-25 represents amino acids 1-25), and the ordinate represents the ratio of the detected OD value to the detected OD value of the untruncated segment.

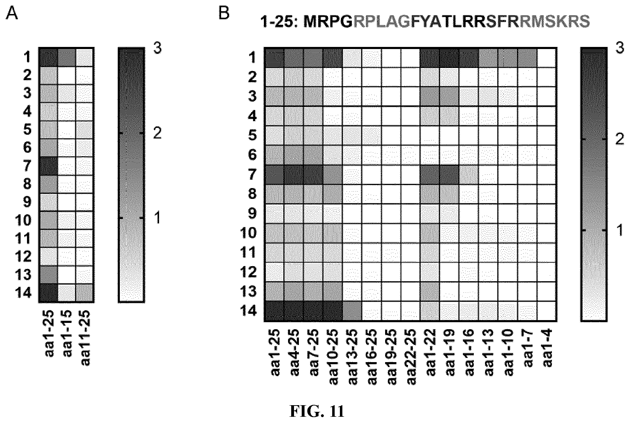

FIG. 11 shows the reactivity of serum samples of nasopharyngeal cancer with different truncated polypeptides within the aa1-25 segment in Example 10. FIG. 11A shows the reactivity of different serum samples of nasopharyngeal cancer patients with aa1-25, aa1-15 and aa11-25, and FIG. 11B shows the reactivity of these serum samples with aa1-25 and its truncated polypeptides. The abscissa represents the amino acid position of the synthetic peptide, and the ordinate represents the number of nasopharyngeal cancer serum, in which the darker the color, the higher the reactivity.

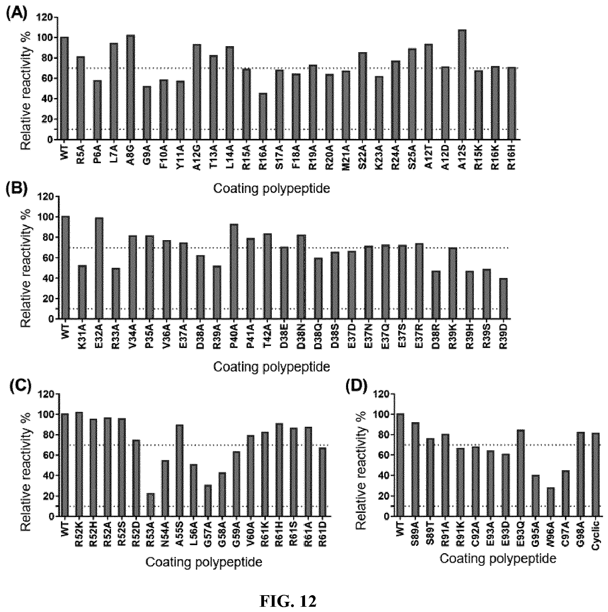

FIG. 12 shows the reactivity of IgG antibody in nasopharyngeal cancer patients with BNLF2b-encoded protein mutants in Example 10, in which A to D represent the reactivity of serum samples of nasopharyngeal cancer with mutants within aa1-25 segment, mutants within aa31-45 segment, mutants within aa51-65 segment and mutants within aa81-98 segment, respectively; the abscissa represents mutation position, and the ordinate represents the ratio of the detected OD value to the detected OD value of the corresponding unmutated segment.

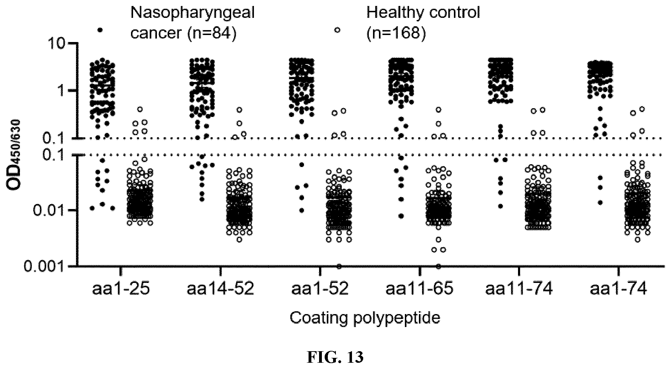

FIG. 13 shows the reactivity of IgG antibody in serum samples of nasopharyngeal cancer patients and healthy controls with different BNLF2b-encoded polypeptides in Example 11, in which the abscissa represents polypeptide fragment, and the ordinate represents the detected OD value.

### Sequence information

**Table 1: Information of sequences referred to in the present application is described in the table below.**

| SEQ ID NO: | Description | SEQ ID NO: | Description |
|---|---|---|---|
| 1 | EBV ORF 1, aa467-486 | 53 | EBV ORF 57, aa326-345 |
| 2 | EBV ORF 2, aa90-109 | 54 | EBV ORF 59, aa55-74 |
| 3 | EBV ORF 3, aa138-157 | 55 | EBV ORF 59, aa231-250 |
| 4 | EBV ORF 15, aa12-31 | 56 | EBV ORF 60, aa195-214 |
| 5 | EBV ORF 16, aa103-122 | 57 | EBV ORF 60, aa82-101 |
| 6 | EBV ORF 16, aa340-359 | 58 | EBV ORF 61, aa18-37 |
| 7 | EBV ORF 17, aa26-45 | 59 | EBV ORF 62, aa41-60 |
| 8 | EBV ORF 18, aa110-129 | 60 | EBV ORF 63, aa280-299 |
| 9 | EBV ORF 19, aa394-413 | 61 | EBV ORF 64, aa88-107 |
| 10 | EBV ORF 19, aa372-391 | 62 | EBV ORF 64, aa154-173 |
| 11 | EBV ORF 20, aa63-82 | 63 | EBV ORF 65, aa118-137 |
| 12 | EBV ORF 20, aa101-120 | 64 | EBV ORF 66, aa88-107 |
| 13 | EBV ORF 21, aa670-689 | 65 | EBV ORF 66, aa359-378 |
| 14 | EBV ORF 22, aa330-349 | 66 | EBV ORF 67, aa244-263 |
| 15 | EBV ORF 23, aa205-224 | 67 | EBV ORF 68, aa120-139 |
| 16 | EBV ORF 26, aa110-129 | 68 | EBV ORF 69, aa17-36 |
| 17 | EBV ORF 27, aa256-275 | 69 | EBV ORF 70, aa171-190 |
| 18 | EBV ORF 28, aa9-28 | 70 | EBV ORF 71, aa201-220 |
| 19 | EBV ORF 29, aa1-20 | 71 | EBV ORF 72, aa143-162 |
| 20 | EBV ORF 30, aa789-801 | 72 | EBV ORF 73, aa195-214 |
| 21 | EBV ORF 31, aa185-204 | 73 | EBV ORF 73, aa154-173 |
| 22 | EBV ORF 32, aa246-265 | 74 | EBV ORF 75, aa514-533 |
| 23 | EBV ORF 33, aa26-45 | 75 | EBV ORF 75, aa432-451 |
| 24 | EBV ORF 34, aa134-153 | 76 | EBV ORF 76, aa149-168 |
| 25 | EBV ORF 34, aa61-80 | 77 | EBV ORF 77, aa180-199 |
| 26 | EBV ORF 35, aa665-684 | 78 | EBV ORF 78, aa19-38 |
| 27 | EBV ORF 35, aa822-841 | 79 | EBV ORF 79, aa323-342 |
| 28 | EBV ORF 36, aa60-79 | 80 | EBV ORF 79, aa579-598 |
| 29 | EBV ORF 37, aa92-111 | 81 | EBV ORF 80, aa32-51 |
| 30 | EBV ORF 38, aa1-20 | 82 | EBV ORF 81, aa11-30 |
| 31 | EBV ORF 39, aa105-124 | 83 | EBV ORF 82, aa155-174 |
| 32 | EBV ORF 39, aa593-612 | 84 | EBV ORF 83, aa66-85 |
| 33 | EBV ORF 40, aa568-587 | 85 | EBV ORF 84, aa188-207 |
| 34 | EBV ORF 41, aa63-82 | 86 | EBV ORF 85, aa31-50 |
| 35 | EBV ORF 41, aa177-196 | 87 | EBV ORF 86, aa19-38 |
| 36 | EBV ORF 42, aa179-198 | 88 | EBV ORF 85, aa14-52 |
| 37 | EBV ORF 42, aa1-20 | 89 | EBV ORF 85, aa1-74 |
| 38 | EBV ORF 43, aa444-463 | 90 | EBV ORF 85, aa14-74 |
| 39 | EBV ORF 44, aa126-145 | 91 | EBV ORF 85, aa1-52 |
| 40 | EBV ORF 44, aa2-21 | 92 | EBV ORF 85, aa1-15 |
| 41 | EBV ORF 45, aa495-514 | 93 | EBV ORF 85, aa11-25 |
| 42 | EBV ORF 45, aa10-29 | 94 | EBV ORF 85, aa21-35 |
| 43 | EBV ORF 47, aa60-79 | 95 | EBV ORF 85, aa31-45 |
| 44 | EBV ORF 48, aa212-231 | 96 | EBV ORF 85, aa41-55 |
| 45 | EBV ORF 50, aa45-64 | 97 | EBV ORF 85, aa51-65 |
| 46 | EBV ORF 52, aa211-230 | 98 | EBV ORF 85, aa61-75 |
| 47 | EBV ORF 53, aa166-185 | 99 | EBV ORF 85, aa71-85 |
| 48 | EBV ORF 53, aa1-20 | 100 | EBV ORF 85, aa81-98 |
| 49 | EBV ORF 54, aa209-228 | 101 | BNLF2b gene-encoded full-length protein |
| 50 | EBV ORF 54, aa104-123 | 102 | EBV ORF 85, aa1-25 |
| 51 | EBV ORF 55, aa357-376 | 103 | EBV ORF 85, aa11-65 |
| 52 | EBV ORF 56, aa50-69 | 104 | EBV ORF 85, aa11-74 |

### Examples

### Example 1: Synthesis of Epstein-Barr virus gene-encoded polypeptides

Based on the amino acid sequence information of the proteins encoded by the 86 open reading frames (ORFs) of the EBV B95-8 strain in GenBank (GenBank ID: V01555.2), the B cell epitopes of each protein were predicted online by bioinformatics tools; according to the prediction results, 1 to 2 possible B cell epitope peptides were selected for each protein and entrusted to Xiamen Jingju Biotechnology Co., Ltd. for synthesis. During synthesis, biotin was coupled to the N-terminus of polypeptide to facilitate subsequent experiments. Finally, 87 Epstein-Barr virus gene-encoded polypeptides (SEQ ID NOs: 1 to 87) were successfully synthesized, and these polypeptides were derived from 68 ORFs, and the specific information thereof was shown in Table 1.

### Example 2: Evaluation of reactivity of EBV polypeptides with serum IgA antibody

The biotin-labeled polypeptides (Nos. 1 to 87) obtained in Example 1 were diluted to 500 ng/ml, respectively, and added to a streptavidin-coated 96-well microwell plate at 100 µL per well, for reaction at 37°C for 2 hours. After the reaction, washing twice with PBST, and 200 µL of blocking solution was added to each well, for blocking for 2 hours at 37 °C. After the blocking, the blocking solution was discarded, and 100 µL of 1:20 diluted nasopharyngeal cancer patient serum or negative control serum was added to each well, and the reaction was performed at 37 °C for 30 minutes. After the reaction, washing 5 times with PBST, and 1:20,000 diluted HRP-labeled goat-anti-human IgA (KPL, Gaithersburg, MD) was added, and the reaction was continued for 30 minutes at 37 °C. After washing 5 times with PBST, 100 µL of TMB chromogenic solution was added to each well; after incubation at 37 °C for 15 minutes, 50 µL of termination solution was added to each well; after mixing, the absorbance at 450 and 630 nm was measured by a microplate reader.

The results were shown in FIG. 1. Among the 87 polypeptides, 8 polypeptides had an OD value above 0.3 for the reaction with nasopharyngeal cancer pooled serum (Table 2-1). Among these 8 polypeptides, 5 polypeptides encoded by BSRL1, BLLF1b, BGLF3, BDLF2 and BVRF2 genes had not been reported for auxiliary diagnosis of nasopharyngeal cancer in the previous studies.

We further evaluated the specificity of these 5 polypeptides through 36 negative serum samples, and the results were shown in FIG. 2 and Table 2-2. The specificity of the 3 polypeptides encoded by BSRF1, BGLF3 and BVRF2 genes was relatively better. At the same time, we evaluated the detection sensitivity of the above 3 polypeptides through 12 serum samples of nasopharyngeal cancer, and the results were shown in FIG. 2. The reactivity of these 3 polypeptides was low, in which the BSRF1- and BVRF2-encoded polypeptides showed reactivity of higher than 0.1 with 6 serum samples, while the BGLF3-encoded polypeptide showed reactivity of higher than 0.1 with only 3 serum samples.

**Table 2-1: Reactivity of EBV polypeptides with nasopharyngeal cancer patient serum IgA antibody**

| SEQ ID No: | Gene | Open reading frame | Corresponding protein | Positive serum OD_{450/630} |
|---|---|---|---|---|
| 21 | BSRF1 | 31 | - | 0.403 |
| 27 | BLLF1b | 35 | gp220 | 0.808 |
| 36 | BZLF1 | 42 | Zta | 0.341 |
| 38 | BRLF1 | 43 | Rta | 0.787 |
| 54 | BGLF3 | 59 | UL14 | 0.382 |
| 65 | BDLF2 | 66 | - | 0.390 |
| 75 | BVRF2 | 75 | Protease | 0.783 |
| 76 | BdRF1 | 76 | VCA-p40 | 0.424 |

**Table 2-2: Reactivity of EBV polypeptides with negative control serum IgA antibody**

| SEQ ID No: | Gene | Negative serum OD_{450/630} | |
|---|---|---|---|
| | | Geometric mean (range) | Ratio of greater than 0.1 |
| 21 | BSRF1 | 0.027 (0.001, 0.126) | 1/36 |
| 27 | BLLF1b | 0.194 (0.031, 2.54) | 26/36 |
| 54 | BGLF3 | 0.025 (0.006, 0.164) | 1/36 |
| 65 | BDLF2 | 0.128 (0.036, 1.766) | 19/36 |
| 75 | BVRF2 | 0.036 (0.011,0.128) | 2/36 |

### Example 3: Evaluation of reactivity of EBV polypeptides with serum IgG antibody

The reactivity of 87 polypeptides (1 to 87) synthesized in Example 1 with serum IgG was detected according to the method in Example 2, wherein HRP-labeled mouse anti-human IgG (Wanyu Meilan, Beijing) diluted by 1:5000 was used to replace the goat anti-human IgA. The results were shown in FIG. 1. Among these polypeptides, there were 5 polypeptides encoded by genes such as BZLF1 and BRLF1 that had reactivity higher than 0.1 with the pooled serum of nasopharyngeal cancer (Table 3-1), in which BNLF1 (ZTA), BRLF1 (RTA) and BILF2 (gp78) had been reported.

We further evaluated the specificity of the BVRF2- and BNLF2b-encoded polypeptides through 36 negative serum samples, and the results were shown in FIG. 2 and Table 3-2, in which the OD values for the reaction of the BNLF2b gene-encoded polypeptides with 36 negative serum samples were all lower than 0.034, indicating good specificity. At the same time, we evaluated the detection sensitivity of the BNLF2b gene-encoded polypeptides by 12 nasopharyngeal cancer serum samples, and the results were shown in FIG. 2, in which 6 of the 12 serum samples of nasopharyngeal cancer patient had reaction OD value of higher than 0.1.

**Table 3-1: Reactivity of EBV polypeptides with positive serum IgG antibody**

| SEQ ID No: | Gene | Open reading frame | Corresponding protein | Positive serum OD_{450/630} |
|---|---|---|---|---|
| 36 | BZLF1 | 42 | Zta | 0.367 |
| 38 | BRLF1 | 43 | Rta | 0.334 |
| 75 | BVRF2 | 75 | protease | 0.883 |
| 77 | BILF2 | 77 | gp78 | 0.171 |
| 86 | BNLF2b | 85 | - | 0.539 |

**Table 2-2: Reactivity of EBV polypeptides with negative control serum IgG antibody**

| SEQ ID No: | Gene | Negative serum OD_{450/630} | |
|---|---|---|---|
| | | Geometric mean (range) | Ratio of greater than 0.1 |
| 75 | BVRF2 | 0.072 (0.020,3.139) | 8/36 |
| 86 | BNLF2b | 0.019 (0.011,0.034) | 0/36 |

### Example 4: Establishment and preliminary performance evaluation of indirect method of anti-BNLF2b antibody

We further analyzed the hydrophilicity, hydrophobicity and antigenicity of the protein encoded by BNLF2b (SEQ ID NO: 101) through the Protean software in the DNASTAR software package, and synthesized two polypeptides aa14-52 (SEQ ID NO: 88) and aa1-74 (SEQ ID NO: 89), which were used as coating antigens for indirect detection.

### 4.1 Indirect detection based on polypeptide aa14-52

The polypeptide aa14-52 was diluted to 125 ng/ml with carbonate buffer (pH 9.6), and coating was performed with 100 µL per well. According to the method in Example 3, 86 serum samples of nasopharyngeal cancer patients and 195 serum samples of healthy human were used in the detection. The results were shown in FIG. 3A, in which when aa14-52 was used as the coating antigen, 57 of 86 serum samples of nasopharyngeal cancer patient had OD value of higher than 0.1, and all of 195 serum samples of healthy people had OD value of lower than 0.025. At the same time, the same method was used to detect the serum samples of 122 subjects who were high-risk according to the EBNA1/IgA+VCA/IgA combined screening (see Example 6 for the combined detection method) but ultimately diagnosed as non-nasopharyngeal cancer, and the results were shown in FIG. 3A, which showed that only 3 cases had OD value of higher than 0.1. The ROC curve analysis by MedCalc 16.2.1 software (MedCalc Software, Ostend, Belgium) was performed on the data of the above-mentioned indirect detection based on polypeptide aa14-52, and the results were shown in FIG. 3B, in which the area under curve AUC was 0.942, when the cut-off value was 0.025, the Youden index was 0.80, the specificity was 100%, and the sensitivity was 80.23% (69/86).

### 4.2 Indirect detection based on polypeptide aa1-74

Using the polypeptide aa1-74 as the coating antigen, 63 serum samples of healthy people and 221 serum samples of nasopharyngeal cancer were detected according to the same method as 4.1. As shown in FIG. 4A, 55 of the 63 serum samples of nasopharyngeal cancer patient had a detected OD value of more than 0.1, while only 10 of the 221 serum samples of healthy control had a detected OD value of more than 0.1. The ROC curve analysis of the above-mentioned indirect method based on polypeptide aa1-74 was further performed, and the results were shown in FIG. 4B, which indicated that the method could efficiently distinguish nasopharyngeal cancer patients from healthy people, with an AUC of 0.950. When the cut-off value was set to 0.1, the sensitivity was 87.30% (55/63) and the specificity was 95.48% (211/221).

In addition, the polypeptide aa1-74 was also used as the coating antigen to detect the levels of IgA and IgM antibodies in 221 serum samples of healthy control by the indirect method, in which IgA antibody detection used 1:20000 diluted HRP-labeled goat anti-human IgA (KPL, Gaithersburg, MD), and IgM antibody detection used 1:50000 diluted HRP-labeled goat anti-human IgM (Wanyu Meilan, Beijing). The results were shown in FIG. 4C, which indicated that the detection of anti-BNLF2b IgA antibody and anti-BNLF2b IgM antibody showed good specificity, only 4 and 7 serum samples had a detected value of higher than 0.1, respectively, and the specificity was 98.19% (217/221) and 96.83% (214/221), respectively.

Table 4 showed the performance of the above methods in distinguishing nasopharyngeal cancer and non-nasopharyngeal cancer. The results showed that whether the indirect method of IgG antibody using polypeptide aa14-52 as the coating antigen, or the indirect methods of IgG, IgA and IgM antibodies using polypeptide aa1-74 as the coating antigen, could effectively distinguish nasopharyngeal cancer from healthy control, and had good detection sensitivity and specificity.

**Table 4: Performance of anti-BNLF2b antibody indirect method to distinguish nasopharyngeal cancer from non-nasopharyngeal cancer**

| Coating peptide fragment | Antibody type | AUC | Cut-off | Sensitivity | Specificity |
|---|---|---|---|---|---|
| aa14-52 | IgG | 0.942 | 0.1 | 66.3% | 100% |
| | | | | (57/86) | (195/195) |
| aa14-52 | IgG | 0.942 | 0.025 | 80.23% | 100% |
| | | | | (69/86) | (195/195) |
| aa1-74 | IgG | 0.950 | 0.1 | 87.30% | 95.48% |
| | | | | (55/63) | (211/221) |
| aa1-74 | IgA | - | 0.1 | - | 98.19% |
| | | | | | (217/221) |
| aa1-74 | IgM | - | 0.1 | - | 96.83% |
| | | | | | (214/221) |

### Example 5: Establishment of double-antigen sandwich method for anti-BNLF2b antibody

Since the results of Example 4 showed that the 3 kinds of antibodies IgA, IgM and IgG all had good specificity in predicting the risk of nasopharyngeal cancer, the risk of nasopharyngeal cancer could be predicted by detecting the total antibody against BNLF2b. Based on this, in this example, a double-antigen sandwich method for detecting anti-BNLF2b antibody was established to evaluate its performance in screening nasopharyngeal cancer.

The BNLF2b-encoded polypeptide aa1-74 was diluted to 100 ng/mL with carbonate buffer (pH 9.6), added to a standard 96-well microwell plate at 100 µL per well, and reacted at 37 °C for 2 hours. After the reaction, washing twice with PBST, and 200 µL of blocking solution was added to each well, and the blocking was performed at 37 °C for 2 hours. After blocking, the blocking solution was discarded, and 50 µL of dilution containing 67 ng/ml biotinylated polypeptide aa1-74 and 50 µL of nasopharyngeal cancer patient serum or negative control serum were added to each well, and the reaction was performed at 37 °C for 60 minutes. After the reaction, washing 5 times with PBST, and 1:5000 diluted HRP-labeled streptavidin and 1:15000 diluted HRP-labeled aa1-74 were added, and the reaction was continued for 30 minutes at 37 °C. After washing 5 times with PBST, 100 µL of TMB chromogenic solution was added to each well; after incubation at 37 °C for 15 minutes, 50 µL of termination solution was added to each well; after mixing, the absorbance at 450 nm and 630 nm was measured by a microplate reader. This method was used to detect 50 serum samples of nasopharyngeal cancer patient and 500 serum samples of healthy people, and the results were shown in FIG. 5A. 46 of the 50 serum samples of nasopharyngeal cancer patient had a reaction OD value of higher than 0.1, while only 1 of 500 negative serum samples had OD value of higher than 0.1. The above results were further subjected to ROC curve analysis, and the results were shown in FIG. 5B, in which the area under curve was 0.977, and when the cut-off was 0.1, the sensitivity and specificity of the reagent were 92.0% and 99.8%, respectively, and the Youden index was 0.91.

In addition, in order to compare the performance of different BNLF2b peptides in the diagnosis of nasopharyngeal cancer, in addition to aa1-74, we also synthesized two peptides aa1-52 (SEQ ID NO: 91) and aa14-74 (SEQ ID NO: 90), and both of them were labeled with biotin at C-terminus. Another 175 serum samples of nasopharyngeal cancer patient were taken and detected according to the same method. The results were shown in the following table. When the cut-off was 0.1, the sensitivity of all of the three polypeptides were higher than 85%, indicating that the method had high detection sensitivity and specificity by using aa1-52, aa14-74 or aa1-74 as the coating antigen.

**Table 5: Comparison of detection sensitivity when using different BNLF2b polypeptides as labeled antigen**

| Coating peptide | Mean absorbance | 95% Confidence interval | Cut-off | Number of positives | Sensitivity |
|---|---|---|---|---|---|
| aa1-74 | 0.604 | 0.008-2.422 | 0.1 | 160 | 91.43% |
| aa1-52 | 0.575 | 0.009-2.809 | 0.1 | 155 | 88.57% |
| aa14-74 | 0.422 | 0.007-1.694 | 0.1 | 155 | 88.57% |

### Example 6: Comparison of the double-antigen sandwich method for anti-BNLF2b antibody and existing nasopharyngeal cancer screening reagents

Parallel detection of 74 serum samples of nasopharyngeal cancer patient and 250 serum samples of healthy people were performed by using the EBNA1/IgA detection kit of Zhongshan Bio, the VCA/IgA detection kit of EU (Cat. No.: EI 2791-9601A) and the double-antigen sandwich method of Example 5 (aa1-74). The detection results of EBNA1/IgA were shown in FIG. 6A, and the detection results of VCA/IgA were shown in FIG. 6B. Usually, EBNA1/IgA and VCA/IgA were used in combination as screening indicators, and when the two antibodies were used in combination, the probability of nasopharyngeal cancer was calculated as follows: LogitP = -3.934 + 2.203 × VCA/IgA + 4.797 × EBNA1/IgA (Liu, Z., et al. 2013, Am J Epidemiol), with the cut-off of 0.98. The results of the combined detection of EBNA1/IgA+VCA/IgA were shown in FIG. 6C, which indicated that 69 of the 74 nasopharyngeal cancer patients and 8 of the 250 healthy people had a probability of higher than 0.98, and the sensitivity and specificity were 93.64 % and 96.80%, respectively.

The detection results of anti-BNLF2b antibody were shown in FIG. 7, in which 70 of serum samples of nasopharyngeal cancer patient and 1 serum sample of healthy people had an OD value of higher than 0.1, and when 0.1 was used as the cut-off value, the sensitivity and specificity of the anti-BNLF2b antibody detection were 94.59% and 99.60%, respectively. The results were further analyzed by chi-square test using SPSS software, and it was found that detection of anti-BNLF2b antibody and the combination of EBNA1/IgA+VCA/IgA showed no significant difference in sensitivity (P=1.00), but anti-BNLF2b antibody showed a specificity significantly higher than that of the combination of EBNA1/IgA+VCA/IgA (P=0.037).

The ROC curve analysis was performed for the results of the above anti-BNLF2b antibody detection and the combination of EBNA1/IgA+VCA/IgA, respectively. The results were shown in FIG. 8, in which the AUC values of the anti-BNLF2b antibody detection and the combination of EBNA1/IgA+VCA/IgA were 0.97 and 0.99, respectively, and there was no significant difference overall (P=0.316), but when the specificity was 94.59% or lower, the anti-BNLF2b antibody detection always showed a sensitivity higher than that of the combined detection. In addition, 28 of the 74 nasopharyngeal cancer patients were in stage I/II; among these 28 patients, one case was false negative in both the BNLF2b detection and the combination of EBNA1/IgA+VCA/IgA, and thus the sensitivity was 96.43%.

The data in Example 5 and Example 6 were combined for calculation, which showed the anti-BNLF2b antibody detection had a sensitivity of 93.55% (116/124) and a specificity of 99.73% (748/750). According to the incidence calculation formula in the literature (Liu, Z., et al. 2013, Am J Epidemiol), the positive predictive value of the anti-BNLF2b antibody detection was 33.2%, which showed a significant improvement as compared with the positive predictive value (4.4%, 38/862) of the EBNA1/IgA+VCA/IgA combined detection (P<0.0001). The above results indicated that the anti-BNLF2b antibody detection could significantly improve the specificity and positive predictive value of nasopharyngeal cancer screening as compared with the existing combination of EBNA1/IgA+VCA/IgA screening method.

### Example 7: Application of anti-BNLF2b antibody double-antigen sandwich method in nasopharyngeal cancer screening

Population screening was carried out in two towns in high-incidence areas in Zhongshan City, Guangdong Province (Fusha and Nanlang), a total of 1325 people were enrolled, 496 people in Fusha and 829 in Nanlang. According to the method in Example 6 (herein after referred to as the anti-BNLF2b antibody detection), these samples were subjected to parallel detections for EBNA1/IgA, VCA/IgA and anti-BNLF2b antibodies; according to the formula combination of EBNA1/IgA and VCA/IgA (LogitP = -3.934 + 2.203 × VCA/IgA + 4.797 × EBNA1/IgA), the probability of developing nasopharyngeal cancer was calculated. The results were shown in the table below, which indicated that among the 1325 people, 163, 218 and 32 people were positive for EBNA1/IgA, VCA/IgA and anti-BNLF2b antibodies, respectively. Among the 126 people with probability index of greater than 0.98, 5 were ultimately diagnosed as nasopharyngeal cancer, while among those with a probability of less than 0.98, none was pre-diagnosed to have nasopharyngeal cancer according to the tumor registry system. Among the 5 samples that were diagnosed as nasopharyngeal cancer, the VCA/IgA could detect only 1 sample, while the other methods could detect 100%. In the 1320 non-nasopharyngeal cancer samples, the specificity and positive predictive value of the anti-BNLF2b antibody detection were 97.95% and 15.63%, respectively, which were higher than those of the EBNA1/IgA, VCA/IgA and their combination.

**Table 6: Comparison of performance of anti-BNLF2b antibody and existing markers in population screening**

| Screening protocol | sensitivity (n=5) | Specificity (n=1320) | Positive predictive value | Negative predictive value |
|---|---|---|---|---|
| EBNA1/IgA | 100% | 88.03% | 3.07% | 100.00% |
| | (5/5) | (1162/1320) | (5/163) | (1162/1162) |
| VCA/IgA | 20% | 83.56% | 0.46% | 99.64% |
| | (1/5) | (1103/1320) | (1/218) | (1103/1107) |
| Probability | 100% | 90.83% | 3.97% | 100.00% |
| (EBNA1/IgA+VCA/IgA) | (5/5) | (1199/1320) | (5/126) | (1199/1199) |
| Anti-BNLF2b antibody | 100% | 97.95% | 15.63% | 100.00% |
| | (5/5) | (1293/1320) | (5/32) | (1293/1293) |

### Example 8: Combined use of anti-BNLF2b antibody double-antigen sandwich method and existing nasopharyngeal cancer screening reagent

The data in Example 6 and Example 7 were combined, and there were 79 cases of nasopharyngeal cancer and 1570 cases of non-nasopharyngeal cancer. The detection results of 79 nasopharyngeal cancer samples were shown in Table 7-1, in which there were 70, 67 and 72 cases that were positive in the anti-BNLF2b antibody detection were also positive in EBNA1/IgA, VCA/IgA and probability, respectively. The detection results of the 1570 non-nasopharyngeal cancer cases were shown in Table 7-2, in which there were only 5, 5 and 4 cases that were positive in the anti-BNLF2b antibody detection were also positive in EBNA/IgA, VCA/IgA and probability, respectively. The results of the further combined detection of the anti-BNLF2b antibody detection with EBNA/IgA, VCA/IgA, EBNA/IgA+VCA/IgA were shown in Table 7-3, which indicated that after combined detection with EBNA/IgA, the positive predictive value increased from 15.15% to 50%, and after combined detection with the probability, the positive predictive value increased from 15.15% to 55.56%.

**Table 7-1: Detection results of 79 nasopharyngeal cancer samples**

| Anti-BNLF2b | EBNA1/IgA | | | VCA/IgA | | | Probability | |
|---|---|---|---|---|---|---|---|---|
| | Positive | Negative | | Positive | Negative | | >0.98 | ≦0.98 |
| Positive | 70 | 5 | | 67 | 8 | | 72 | 3 |
| Negative | 3 | 1 | | 2 | 2 | | 2 | 2 |

**Table 7-2: Detection results of 1570 non-nasopharyngeal cancer samples**

| Anti-BNLF2b | EBNA1/IgA | | | VCA/IgA | | | Probability | |
|---|---|---|---|---|---|---|---|---|
| | Positive | Negative | | Positive | Negative | | >0.98 | ≦0.98 |
| Positive | 5 | 23 | | 5 | 23 | | 4 | 24 |
| Negative | 153 | 1389 | | 228 | 1314 | | 125 | 1417 |

**Table 7-3: Performance of anti-BNLF2b antibody detection and combined detection with existing markers for nasopharyngeal cancer screening**

| Performance | Anti-BNLF2b antibody detection | | | |
|---|---|---|---|---|
| | - | EBNA1/IgA | VCA/IgA | Probability |
| Sensitivity | 94.94% | 88.61% | 84.81% | 91.14% |
| Specificity | 98.22% | 99.68% | 99.68% | 99.75% |
| Positive predictive value | 15.15% (5/32) | 50% (5/10) | 16.67% (1/6) | 55.56% (5/9) |
| Negative predictive value | 100% | 100% | 99.75% | 100% |

We further collected 227 cases (Sample II) that were identified as high-risk in the preliminary screening cohort by the combination of EBNA1/IgA+VCA/IgA of Zhongshan People's Hospital, among which 8 were diagnosed as nasopharyngeal cancer by nasopharyngeal endoscopy. The results were shown in Table 8, which indicated that there were 24 cases of anti-BNLF2b antibody positive, 7 of which were nasopharyngeal cancer, and thus the sensitivity and positive predictive value of the combined detection were 87.50% and 29.17%, respectively.

Taken Sample II and Sample I together, wherein Sample I included 8 cases with probability >0.98 in the 250 healthy controls in Example 6, and 126 cases (5 cases had nasopharyngeal cancer) with probability >0.98 in the screening cohort of 1325 people in Example 7. After merging the two samples, a total of 13 nasopharyngeal cancer cases and 348 non-nasopharyngeal cancer cases were obtained, and the positive predictive value of the combination of EBNA1/IgA+VCA/IgA was 3.60%. On this basis, after the anti-BNLF2b antibody detection, 12 of the 13 nasopharyngeal cancer cases were positive, while only 21 of the 348 non-nasopharyngeal cancers were positive, and thus the positive predictive value of the combined detection was increased to 36.36% (Table 8). Therefore, the combined detection of anti-BNLF2b antibody and EBNA1/IgA+VCA/IgA could further improve the specificity and positive predictive value of nasopharyngeal cancer screening. In order to reduce the workload, anti-BNLF2b antibodies could be detected first, followed by further detection of EBNA1/IgA and VCA/IgA.

**Table 8: Performance of anti-BNLF2b antibody detection in high-risk population**

| Sample | Total number | Number of nasopharyngeal cancer patients | Sensitivity | Specificity | Positive predictive value |
|---|---|---|---|---|---|
| I | 134 | 5 | 100% (5/5) | 96.90% (125/129) | 55.56% (5/9) |
| II | 227 | 8 | 87.50% (7/8) | 92.24% (202/219) | 29.17% (7/24) |
| Total | 361 | 13 | 92.31% (12/13) | 94.24% (327/348) | 36.36% (12/33) |

### Example 9: Application of anti-BNLF2b antibody double-antigen sandwich method in auxiliary diagnosis of nasopharyngeal cancer

The clinical symptoms of nasopharyngeal cancer are not specific enough to distinguish it from other head and neck diseases. Clinically, suspected cases are mainly diagnosed by nasopharyngeal endoscopy and pathological examination. We collected 63 suspected nasopharyngeal cancer cases, of which 31 cases were ultimately diagnosed as nasopharyngeal cancer. We detected the samples of these 63 cases by the double-antigen sandwich method according to the method in Example 6, the results showed that with a cut-off value of 0.1, 30 of the 31 nasopharyngeal cancer cases were positive for BNLF2b antibody, while only 2 of the 32 non-nasopharyngeal cancer cases were positive for BNLF2b antibody. The BNLF2b antibody detection showed a sensitivity of 96.78% (30/31), a specificity of 93.75% (30/32), and a positive predictive value of 93.75% (30/32). The above results indicated that in outpatient cases, the BNLF2b antibody detection could significantly reduce the number of unnecessary nasopharyngeal endoscopy examinations, thereby reducing the economic and physical burden of patients.

### Example 10: Study of immunodominant epitope of BNLF2b-encoded protein

The BNLF2b gene encodes a total of 98 amino acids (SEQ ID NO: 101). In order to analyze the immunodominant epitopes of the protein encoded by BNLF2b, we designed 9 overlapping polypeptides. Each polypeptide had 15 amino acids (the last polypeptide had 18 amino acids), and the two adjacent polypeptides were overlapped by 5 amino acids, and the C-terminal was labeled with biotin for subsequent detection (SEQ ID NOs: 92-100). According to the method in Example 3, by using the above-mentioned polypeptide fragment as the coating antigen, the detection of IgG in 1:300 diluted serum sample of nasopharyngeal cancer patient was performed. The results were shown in FIG. 9, which indicated that the epitopes of the BNLF2b-encoded protein were mainly located in four segments: aa1-25, aa31-45, aa51-65 and aa81-98.

We further preliminarily evaluated the sensitivity of the four segment polypeptides, aa1-25 (SEQ ID NO: 102), aa31-45 (SEQ ID NO: 95), aa51-65 (SEQ ID NO: 97), aa81-98 (SEQ ID NO: 100) in nasopharyngeal cancer screening through 43 serum samples of nasopharyngeal cancer according to the method in Example 3. The results showed that when the cut-off was 0.1, the detection sensitivity was 76.7% (33/43) for aa51-65, 72.1% (31/43) for aa1-25, and the sensitivity of the other two peptides was 55.8% (24/43). The sensitivity of aa14-52 and aa1-74 were 90.7% (39/43) and 93.0% (40/43); and for the 3 serum samples failed in aa1-74 detection, all of the 4 polypeptides were negative, while the reaction of the serum sample of aa14-52 negative and aa1-74 positive with aa51-65 had an OD value of 0.393.

In order to further determine the key amino acids constituting these epitopes, we further performed N-terminal and C-terminal truncations on the basis of aa1-25, aa31-45, aa51-65 and aa81-98, and synthesized a series of polypeptides. In this experiment, 40 serum samples of nasopharyngeal cancer were mixed to form two samples of pooled serum, and after 1:300 dilution, IgG detection was performed according to the method in Example 3, and the results were shown in FIG. 10.
(1) aa1-25 segment (FIG. 10A): when the C-terminus remained unchanged and the N-terminus was truncated to position 16, it could still bind to IgG in nasopharyngeal cancer serum, but when it was truncated to position 19, it completely lost the reactivity with IgG in nasopharyngeal cancer serum; similarly, when the N-terminus remained unchanged and the C-terminus was truncated to position 7, the polypeptide could still reacted with IgG in nasopharyngeal cancer serum, but when the C-terminus was truncated to position 4, it completely lost the reactivity with IgG in nasopharyngeal cancer serum. Therefore, aa1-15 and aa11-25 contained two independent epitopes with key amino acids aa5-7 and aa16-18, respectively.
(2) aa31-45 segment (FIG. 10B): Whether it had N-terminal truncation or C-terminal truncation, the polypeptide completely lost the reactivity with IgG in nasopharyngeal cancer serum without the amino acids EDR at positions 37-39. Therefore, KER (aa31-33) and EDR (aa37-39) were the key amino acids constituting this epitope.
(3) aa51-65 segment (Fig. 10C): When the N-terminus did not contain aa54-56 or the C-terminus did not contain aa60, the polypeptide completely lost the reactivity with nasopharyngeal cancer serum.
(4) aa81-98 segment (Fig. 10D): When the N-terminus did not contain aa87-89 or the C-terminus did not contain aa96-98, the polypeptide completely lost the reactivity with nasopharyngeal cancer serum.

In order to analyze whether the aa1-25 segment contained an epitope spanning the aa1-15 and aa11-25 segments, we detected IgG in 43 serum samples of nasopharyngeal cancer by using aa1-15, aa11-25 and aa1-25 as coating antigens, respectively. The results were shown in FIG. 11A, and indicated that 14 serum samples had an OD value for the reaction with aa1-25 that was higher than the sum of the values for the reaction with aa1-15 and aa11-25. The 14 serum samples were further detected by using different truncated polypeptides in the aa1-25 segment as coating antigens, and the results were shown in FIG. 11B, which indicated that the polypeptides that could react with the positive serum contained at least aa10-16.

In addition, for the above four key segments, we also synthesized a series of variants, including natural and artificial variants. According to the method in Example 3, these variants were used as coating antigens to detect their reactivity with IgG in 1:300 diluted pooled serum sample of nasopharyngeal cancer, and the results were shown in FIG. 12.
(1) aa1-25 (FIG. 12A): Most obvious decrease was observed after mutations of amino acids at positions 6, 9-11 and 16; there were 3 natural mutations at position 12, which were T, D and S, and the mutations of alanine into these 3 amino acids would not significantly decrease the reactivity of aa1-25 with nasopharyngeal cancer serum; in addition, the amino acid mutations at positions 5, 7, 8, 13, 14, 15, 19, 22, 24 and 25 showed relatively little influence.
(2) aa31-45 (FIG. 12B): After K31, R33 as well as D38 and R39 were mutated to alanine, the reactivity of the polypeptide with nasopharyngeal cancer serum decreased significantly, indicating that these amino acids were the key amino acids constituting the epitope; in addition, the amino acid mutations at positions 32, 34, 35, 36, 37, 40, 41, and 42 showed relatively little influence.
(3) aa51-65 (Fig. 12C): When R53 and N54 as well as aa56-59 were mutated to alanine, the reactivity of the polypeptide with nasopharyngeal cancer serum decreased significantly, while the amino acid mutations at positions 52, 55, 60 and 61 showed relatively little influence.
(4) aa81-98 (Fig. 12D): When the three amino acids of aa95-97 were mutated to alanine, the reactivity of the polypeptide with nasopharyngeal cancer serum decreased most obviously; in addition, the amino acid mutations at positions 89, 91, 93 and 98 showed relatively little influence.

The above results showed that the aa5-11, aa16-23, aa31-33, aa37-39, aa53-60 and aa89-98 of the BNLF2b-encoded protein were core segments.

### Example 11: Effect of combined use of different epitopes of BNLF2b-encoded protein on nasopharyngeal cancer screening performance

On the basis of the synthesized polypeptides of aa14-52, aa1-74, aa1-52 and aa1-25 above, another two polypeptides aa11-65 (SEQ ID NO: 103) and aa11-74 (SEQ ID NO: 104) were further synthesized, and labeled with biotin at their C-terminus. According to the method in Example 3, the reaction of these polypeptides with 84 serum samples of nasopharyngeal cancer patient and 168 serum samples of healthy people was detected, respectively. The reactivity of the 6 polypeptides with the serum samples of nasopharyngeal cancer was significantly higher than that with the healthy controls (FIG. 13). The results of ROC curve analysis by Medcal software showed that all of the 6 polypeptides had an area under curve of above 0.95, a sensitivity of above 89% and a specificity of above 96% (Table 9). The above results indicated that the BNLF2b-encoded polypeptide fragments containing one or more of core segments (aa5-11, aa16-23, aa31-33, aa37-39, aa53-60) had excellent screening performance for nasopharyngeal cancer.

**Table 9: Comparison of performance of different polypeptides as capture antigens in nasopharyngeal cancer screening**

| Coating polypeptide | Geometric mean (95% CI) | | ROC curve analysis | | | | |
|---|---|---|---|---|---|---|---|
| | Nasopharyngeal cancer (n=84) | Healthy control (n=168) | AUC | Cut-off | Sensitivity | Specificity | Youden index |
| aa1-25 | 0.646 (0.014-3.897) | 0.016 (0.007-0.125) | 0.968 | 0.071 | 89.29% | 96.43% | 0.857 |
| aa14-52 | 0.758 (0.030-4.446) | 0.011 (0.004-0.054) | 0.990 | 0.054 | 94.05% | 97.62% | 0.917 |
| aal-52 | 1.112 (0.026-4.462) | 0.011 (0.004-0.051) | 0.989 | 0.052 | 95.24% | 97.62% | 0.929 |
| aa11-65 | 1.236 (0.029-4.464) | 0.011 (0.005-0.058) | 0.986 | 0.058 | 94.05% | 97.62% | 0.917 |
| aa11-74 | 1.484 (0.032-4.463) | 0.013 (0.005-0.061) | 0.990 | 0.061 | 95.24% | 97.62% | 0.929 |
| aa1-74 | 1.724 (0.045-3.911) | 0.013 (0.005-0.072) | 0.992 | 0.072 | 96.43% | 97.62% | 0.941 |

Although the specific embodiments of the present invention have been described in detail, those skilled in the art will appreciate that various modifications and changes can be made to the details in light of all the teachings that have been published, and that these changes are all within the scope of the present invention. The full division of the present invention is given by the appended claims and any equivalents thereof.

### References:

[1] Torre LA, Bray F, Siegel RL, Ferlay J, Lortet-Tieulent J, Jemal A. Global cancer statistics, 2012. CA Cancer J Clin. 2015;65:87-108.
[2] Tsao SW, Yip YL, Tsang CM, Pang PS, Lau VM, Zhang G, et al. Etiological factors of nasopharyngeal carcinoma. Oral Oncol. 2014;50:330-8.
[3] Lee AW, Foo W, Law SC, Poon YF, Sze WM, O SK, et al. Nasopharyngeal carcinoma: presenting symptoms and duration before diagnosis. Hong Kong medical journal = Xianggang yi xue za zhi. 1997;3:355-61.
[4] Henle G, Henle W. Epstein-Barr virus-specific IgA serum antibodies as an outstanding feature of nasopharyngeal carcinoma. International journal of cancer. 1976;17:1-7.
[5] Zhu XX, Zeng Y, Wolf H. Detection of IgG and IgA antibodies to Epstein-Barr virus membrane antigen in sera from patients with nasopharyngeal carcinoma and from normal individuals. International journal of cancer. 1986;37:689-91.
[6] Zeng J, Gong CH, Jan MG, Fun Z, Zhang LG, Li HY. Detection of Epstein-Barr virus IgA/EA antibody for diagnosis of nasopharyngeal carcinoma by immunoautoradiography. International journal of cancer. 1983;31:599-601.
[7] Chen JY, Liu MY, Hsu TY, Cho SM, Yang CS. Use of bacterially-expressed antigen for detection of antibodies to the EBV-specific deoxyribonuclease in sera from patients with nasopharyngeal carcinoma. Journal of virological methods. 1993;45:49-66.
[8] Connolly Y, Littler E, Sun N, Chen X, Huang PC, Stacey SN, et al. Antibodies to Epstein-Barr virus thymidine kinase: a characteristic marker for the serological detection of nasopharyngeal carcinoma. International journal of cancer. 2001;91:692-7.
[9] Zhang X, Zhang Y, Nie Y, Wang S, Chen Y, Sun D. Serum Zta antibody of Epstein-Barr virus exerts potential function in the diagnosis of nasopharyngeal cancer. Tumour biology : the journal of the International Society for Oncodevelopmental Biology and Medicine. 2014;35:6879-86.
[10] Littler E, Newman W, Arrand JR. Immunological response of nasopharyngeal carcinoma patients to the Epstein-Barr-virus-coded thymidine kinase expressed in Escherichia coli. International journal of cancer. 1990;45:1028-32.
[11] Gu AD, Lu LX, Xie YB, Chen LZ, Feng QS, Kang T, et al. Clinical values of multiple Epstein-Barr virus (EBV) serological biomarkers detected by xMAP technology. Journal of translational medicine. 2009;7:73.
[12] Wout M. J. van Grunsven WJMS, and Jaap M. Middeldorp. Localization and Diagnostic Application of Immunodominant Domains of the BFRF3-Encoded Epstein-Barr Virus Capsid Protein. The Journal of infectious diseases. 1994;170:13-9.
[13] Zong YS, Sham JS, Ng MH, Ou XT, Guo YQ, Zheng SA, et al. Immunoglobulin A against viral capsid antigen of Epstein-Barr virus and indirect mirror examination of the nasopharynx in the detection of asymptomatic nasopharyngeal carcinoma. Cancer. 1992;69:3-7.
[14] Zeng Y, Zhong JM, Li LY, Wang PZ, Tang H, Ma YR, et al. Follow-up studies on Epstein-Barr virus IgA/VCA antibody-positive persons in Zangwu County, China. Intervirology. 1983;20:190-4.
[15] Zeng Y, Zhang LG, Li HY, Jan MG, Zhang Q, Wu YC, et al. Serological mass survey for early detection of nasopharyngeal carcinoma in Wuzhou City, China. International journal of cancer. 1982;29:139-41.
[16] Chan KH, Gu YL, Ng F, Ng PS, Seto WH, Sham JS, et al. EBV specific antibody-based and DNA-based assays in serologic diagnosis of nasopharyngeal carcinoma. International journal of cancer. 2003;105:706-9.
[17] Chen H, Chi P, Wang W, Li L, Luo Y, Fu J, et al. Evaluation of a semi-quantitative ELISA for IgA antibody against Epstein-Barr virus capsid antigen in the serological diagnosis of nasopharyngeal carcinoma. International journal of infectious diseases : IJID : official publication of the International Society for Infectious Diseases. 2014;25:110-5.
[18] Tang JW, Rohwader E, Chu IM, Tsang RK, Steinhagen K, Yeung AC, et al. Evaluation of Epstein-Barr virus antigen-based immunoassays for serological diagnosis of nasopharyngeal carcinoma. Journal of clinical virology : the official publication of the Pan American Society for Clinical Virology. 2007;40:284-8.
[19] Paramita DK, Fachiroh J, Haryana SM, Middeldorp JM. Two-step Epstein-Barr virus immunoglobulin A enzyme-linked immunosorbent assay system for serological screening and confirmation of nasopharyngeal carcinoma. Clinical and vaccine immunology : CVI. 2009;16:706-11.
[20] Coghill AE, Hsu WL, Pfeiffer RM, Juwana H, Yu KJ, Lou PJ, et al. Epstein-Barr virus serology as a potential screening marker for nasopharyngeal carcinoma among high-risk individuals from multiplex families in Taiwan. Cancer epidemiology, biomarkers & prevention : a publication of the American Association for Cancer Research, cosponsored by the American Society of Preventive Oncology. 2014;23:1213-9.
[21] Coghill AE, Bu W, Nguyen H, Hsu WL, Yu KJ, Lou PJ, et al. High Levels of Antibody that Neutralize B-cell Infection of Epstein-Barr Virus and that Bind EBV gp350 Are Associated with a Lower Risk of Nasopharyngeal Carcinoma. Clinical cancer research : an official journal of the American Association for Cancer Research. 2016;22:3451-7.
[22] Chang C, Middeldorp J, Yu KJ, Juwana H, Hsu WL, Lou PJ, et al. Characterization of ELISA detection of broad-spectrum anti-Epstein-Barr virus antibodies associated with nasopharyngeal carcinoma. Journal of medical virology. 2013;85:524-9.
[23] Li RC, Du Y, Zeng QY, Tang LQ, Zhang H, Li Y, et al. Epstein-Barr virus glycoprotein gH/gL antibodies complement IgA-viral capsid antigen for diagnosis of nasopharyngeal carcinoma. Oncotarget. 2016;7:16372-83.
[24] Fachiroh J, Paramita DK, Hariwiyanto B, Harijadi A, Dahlia HL, Indrasari SR, et al. Single-assay combination of Epstein-Barr Virus (EBV) EBNA1- and viral capsid antigen-p 18-derived synthetic peptides for measuring anti-EBV immunoglobulin G (IgG) and IgA antibody levels in sera from nasopharyngeal carcinoma patients: options for field screening. Journal of clinical microbiology. 2006;44:1459-67.
[25] Cai YL, Li J, Lu AY, Zheng YM, Zhong WM, Wang W, et al. Diagnostic significance of combined detection of Epstein-Barr virus antibodies, VCA/IgA, EA/IgA, Rta/IgG and EBNA1/IgA for nasopharyngeal carcinoma. Asian Pacific journal of cancer prevention : APJCP. 2014;15:2001-6.
[26] Liu Y, Huang Q, Liu W, Liu Q, Jia W, Chang E, et al. Establishment of VCA and EBNA1 IgA-based combination by enzyme-linked immunosorbent assay as preferred screening method for nasopharyngeal carcinoma: a two-stage design with a preliminary performance study and a mass screening in southern China. International journal of cancer. 2012;131:406-16.
[27] Ji MF, Wang DK, Yu YL, Guo YQ, Liang JS, Cheng WM, et al. Sustained elevation of Epstein-Barr virus antibody levels preceding clinical onset of nasopharyngeal carcinoma. British journal of cancer. 2007;96:623-30.
[28] Ji MF, Yu YL, Cheng WM, Zong YS, Ng PS, Chua DT, et al. Detection of Stage I nasopharyngeal carcinoma by serologic screening and clinical examination. Chinese journal of cancer. 2011;30:120-3.
[29] Liu Z, Ji MF, Huang QH, Fang F, Liu Q, Jia WH, et al. Two Epstein-Barr virus-related serologic antibody tests in nasopharyngeal carcinoma screening: results from the initial phase of a cluster randomized controlled trial in Southern China. American journal of epidemiology. 2013;177:242-50.
[30] Li T, Guo X, Ji M, Li F, Wang H, Cheng W, et al. Establishment and validation of a two-step screening scheme for improved performance of serological screening of nasopharyngeal carcinoma. Cancer Med. 2018;7:1458-67.
[31] Ji MF, Huang QH, Yu X, Liu Z, Li X, Zhang LF, et al. Evaluation of plasma Epstein-Barr virus DNA load to distinguish nasopharyngeal carcinoma patients from healthy high-risk populations in Southern China. Cancer. 2014;120:1353-60.
[32] Chen Y, Zhao W, Lin L, Xiao X, Zhou X, Ming H, et al. Nasopharyngeal Epstein-Barr Virus Load: An Efficient Supplementary Method for Population-Based Nasopharyngeal Carcinoma Screening. PloS one. 2015;10:e0132669.
[33] Zhang G, Zong J, Lin S, Verhoeven RJ, Tong S, Chen Y, et al. Circulating Epstein-Barr virus microRNAs miR-BART7 and miR-BART13 as biomarkers for nasopharyngeal carcinoma diagnosis and treatment. International journal of cancer. 2015;136:E301-12.
[34] Chan KCA, Woo JKS, King A, Zee BCY, Lam WKJ, Chan SL, et al. Analysis of Plasma Epstein-Barr Virus DNA to Screen for Nasopharyngeal Cancer. The New England journal of medicine. 2017;377:513-22.
[35] Rao Dongping, Liu Qing, Cao Sumei. Cost-effectiveness evaluation of nasopharyngeal cancer screening using Markov model. Chinese Journal of Oncology. 2012;34:549-53.

## Claims

1. An isolated polypeptide or variant thereof, wherein the polypeptide is composed of at least 7 contiguous amino acid residues of a wild-type protein encoded by BNLF2b gene, and comprises at least 1 (e.g., at least 2, at least 3, or all 4) sequence selected from the following: amino acid residues 5-11, amino acid residues 16-23, amino acid residues 31-39, or amino acid residues 53-60 of the wild-type protein encoded by BNLF2b gene;
wherein the variant differs from the polypeptide from which it is derived only by a substitution (e.g., conservative substitution or non-conservative substitution) of one or several (e.g., 1, 2, 3, 4, 5, 6, 7, 8, or 9) amino acid residues, and retains a biological function of the polypeptide from which it is derived (e.g., an activity of being recognized and bound by an anti-EBV antibody);
preferably, the wild-type protein encoded by BNLF2b gene has a sequence set forth in SEQ ID NO: 101.

2. The isolated polypeptide or variant thereof according to claim 1, wherein the isolated polypeptide comprises: amino acid residues 53-60, amino acid residues 5-23, amino acid residues 16-39, amino acid residues 31-60, amino acid residues 5-39, amino acid residues 16-60, or amino acid residues 5-60 of the wild-type protein encoded by BNLF2b gene.

3. The isolated polypeptide or variant thereof according to claim 1 or 2, wherein the isolated polypeptide consists of at least 8 contiguous amino acid residues of the wild-type protein encoded by BNLF2b gene, and comprises amino acid residues 53-60 of the wild-type protein encoded by BNLF2b gene;
preferably, the polypeptide comprises amino acid residues 53-61 of the wild-type protein encoded by BNLF2b gene;
preferably, the polypeptide comprises amino acid residues 51-65 of the wild-type protein encoded by BNLF2b gene;
preferably, the amino acid residues 51-65 have a sequence set forth in SEQ ID NO: 97;
preferably, the polypeptide consists of at least 15 contiguous amino acid residues of the wild-type protein encoded by BNLF2b gene.

4. The isolated polypeptide or variant thereof according to claim 1 or 2, wherein the isolated polypeptide consists of at least 19 contiguous amino acid residues of the wild-type protein encoded by BNLF2b gene, and comprises amino acid residues 5-23 of the wild-type protein encoded by BNLF2b gene;
preferably, the polypeptide comprises amino acid residues 1-25 of the wild-type protein encoded by BNLF2b gene;
preferably, the amino acid residues 1-25 have a sequence set forth in SEQ ID NO: 102;
preferably, the polypeptide consists of at least 25 contiguous amino acid residues of the wild-type protein encoded by BNLF2b gene.

5. The isolated polypeptide or variant thereof according to claim 1 or 2, wherein the isolated polypeptide consists of at least 24 contiguous amino acid residues of the wild-type protein encoded by BNLF2b gene, and comprises amino acid residues 16-39 of the wild-type protein encoded by BNLF2b gene;
preferably, the polypeptide comprises amino acid residues 14-52 of the wild-type protein encoded by BNLF2b gene;
preferably, the amino acid residues 14-52 have a sequence set forth in SEQ ID NO: 88;
preferably, the polypeptide consists of at least 39 contiguous amino acid residues of the wild-type protein encoded by BNLF2b gene.

6. The isolated polypeptide or variant thereof according to claim 1 or 2, wherein the isolated polypeptide consists of at least 30 contiguous amino acid residues of the wild-type protein encoded by BNLF2b gene, and comprises amino acid residues 31-60 of the wild-type protein encoded by BNLF2b gene;
preferably, the isolated polypeptide comprises amino acid residues 31-61 of the wild-type protein encoded by BNLF2b gene.

7. The isolated polypeptide or variant thereof according to claim 1 or 2, wherein the isolated polypeptide consists of at least 35 contiguous amino acid residues of the wild-type protein encoded by BNLF2b gene, and comprises amino acid residues 5-39 of the wild-type protein encoded by BNLF2b gene;
preferably, the polypeptide comprises amino acid residues 1-52 of the wild-type protein encoded by BNLF2b gene;
preferably, the amino acid residues 1-52 have a sequence set forth in SEQ ID NO: 91;
preferably, the polypeptide consists of at least 53 contiguous amino acid residues of the wild-type protein encoded by BNLF2b gene.

8. The isolated polypeptide or variant thereof according to claim 1 or 2, wherein the isolated polypeptide consists of at least 45 contiguous amino acid residues of the wild-type protein encoded by BNLF2b gene, and comprises amino acid residues 16-60 of the wild-type protein encoded by BNLF2b gene;
preferably, the polypeptide comprises amino acid residues 16-61 of the wild-type protein encoded by BNLF2b gene;
preferably, the polypeptide comprises amino acid residues 14-74, amino acid residues 11-65, or amino acid residues 11-74 of the wild-type protein encoded by BNLF2b gene;
preferably, the amino acid residues 14-74 have a sequence set forth in SEQ ID NO: 90;
preferably, the amino acid residues 11-65 have a sequence set forth in SEQ ID NO: 103;
preferably, the amino acid residues 11-74 have a sequence set forth in SEQ ID NO: 104;
preferably, the polypeptide consists of at least 61, at least 55 or at least 64 contiguous amino acid residues of the wild-type protein encoded by BNLF2b gene.

9. The isolated polypeptide or variant thereof according to claim 1 or 2, wherein the isolated polypeptide consists of at least 56 contiguous amino acid residues of the wild-type protein encoded by BNLF2b gene, and comprises amino acid residues 5-60 of the wild-type protein encoded by BNLF2b gene;
preferably, the polypeptide comprises amino acid residues 5-61 of the wild-type protein encoded by BNLF2b gene;
preferably, the polypeptide comprises amino acid residues 1-74 of the wild-type protein encoded by BNLF2b gene;
preferably, the amino acid residues 1-74 have a sequence set forth in SEQ ID NO: 89;
preferably, the polypeptide consists of at least 74 contiguous amino acid residues of the wild-type protein encoded by BNLF2b gene.

10. The isolated polypeptide or variant thereof according to any one of claims 1 to 9, wherein the isolated polypeptide consists of a sequence selected from the group consisting of amino acids 51-56, amino acid residues 1-25, amino acid residues 14-52, amino acid residues 1-52, amino acid residues 14-74, amino acid residues 11-65, amino acid residue 11-74, or amino acid residues 1-74 of the wild-type protein encoded by BNLF2b gene.

11. The isolated polypeptide or variant thereof according to any one of claims 1 to 10, wherein the variant does not comprise an amino acid substitution in amino acid positions corresponding to the following positions: 6, 9, 10, 11, 16, 31, 33, 38, 39, 53, 54, 56, 57, 58, 59, 95, 96, 97 with reference to amino acid positions set forth in the wild-type protein encoded by BNLF2b gene.

12. The isolated polypeptide or variant thereof according to any one of claims 1 to 11, wherein the variant comprise an amino acid substitution at one or more (e.g., 1, 2, 3 or 4) amino acid positions corresponding to the following positions: 5, 7, 8, 12, 13, 14, 15, 19, 22, 24, 25, 32, 34, 35, 36, 37, 40, 41, 42, 52, 55, 60, 61, 89, 91, 93 or 98 with reference to amino acid positions set forth in the wild-type protein encoded by BNLF2b gene;
preferably, the variant comprise an amino acid substitution at one or more (e.g., 1, 2, 3 or 4) amino acid positions corresponding to the following positions: 5, 7, 8, 12, 13, 14, 22, 25, 32, 34, 35, 36, 40, 41, 42, 52, 55, 60, 61, 89, 91, 93 or 98 with reference to amino acid positions set forth in the wild-type protein encoded by BNLF2b gene.

13. The isolated polypeptide or variant thereof according to any one of claims 1 to 11, wherein the variant comprises one or several (e.g., 1, 2, 3, or 4) amino acid substitutions selected from the group consisting of: substitution of an amino acid at a position corresponding to position 5 with A, substitution of an amino acid at a position corresponding to position 7 with A, substitution of an amino acid at a position corresponding to position 8 with G, substitution of an amino acid at a position corresponding to position 12 with G, T, D or S, substitution of an amino acid at a position corresponding to position 13 with A, substitution of an amino acid at a position corresponding to position 14 with G, substitution of an amino acid at a position corresponding to position 15 with A, substitution of an amino acid at a position corresponding to position 22 with A, substitution of an amino acid at a position corresponding to position 24 with A, substitution of an amino acid at a position corresponding to position 25 with A, substitution of an amino acid at a position corresponding to position 32 with A, substitution of an amino acid at a position corresponding to position 34 with A, substitution of an amino acid at a position corresponding to position 35 with A, substitution of an amino acid at a position corresponding to position 36 with A, substitution of an amino acid at a position corresponding to position 37 with A, N, Q, S or R, substitution of an amino acid at a position corresponding to position 40 with A, substitution of an amino acid at a position corresponding to position 41 with A, substitution of an amino acid at a position corresponding to position 42 with A, substitution of an amino acid at a position corresponding to position 52 with K, H, A, S or D, substitution of an amino acid at a position corresponding to position 55 with S, substitution of an amino acid at a position corresponding to position 60 with A, substitution of an amino acid at a position corresponding to position 61 with K, H, S or A, substitution of an amino acid at a position corresponding to position 89 with A or T, substitution of an amino acid at a position corresponding to position 91 with A, substitution of an amino acid at a position corresponding to position 93 with Q, substitution of an amino acid at a position corresponding to position 98 with A;
preferably, the variant comprises one or several (e.g., 1, 2, 3, or 4) amino acid substitutions selected from the group consisting of: substitution of an amino acid at a position corresponding to position 5 with A, substitution of an amino acid at a position corresponding to position 7 with A, substitution of an amino acid at a position corresponding to position 8 with G, substitution of an amino acid at a position corresponding to position 12 with G, T, D or S, substitution of an amino acid at a position corresponding to position 13 with A, substitution of an amino acid at a position corresponding to position 14 with G, substitution of an amino acid at a position corresponding to position 22 with A, substitution of an amino acid at a position corresponding to position 25 with A, substitution of an amino acid at a position corresponding to position 32 with A, substitution of an amino acid at a position corresponding to position 34 with A, substitution of an amino acid at a position corresponding to position 35 with A, substitution of an amino acid at a position corresponding to position 36 with A, substitution of an amino acid at a position corresponding to position 40 with A, substitution of an amino acid at a position corresponding to position 41 with A, substitution of an amino acid at a position corresponding to position 42 with A, substitution of an amino acid at a position corresponding to position 52 with K, H, A, S or D, substitution of an amino acid at a position corresponding to position 55 with S, substitution of an amino acid at a position corresponding to position 60 with A, substitution of an amino acid at a position corresponding to position 61 with K, H, S or A, substitution of an amino acid at a position corresponding to position 89 with A or T, substitution of an amino acid at a position corresponding to position 91 with A, substitution of an amino acid at a position corresponding to position 93 with Q, substitution of an amino acid at a position corresponding to position 98 with A.

14. The isolated polypeptide or variant thereof according to any one of claims 1 to 13, wherein the isolated polypeptide consists of not more than 97 (e.g., not more than 96, not more than 95, not more than 94, not more than 93, not more than 92, not more than 91, not more than 90, not more than 89, not more than 88, not more than 87, not more than 86, not more than 85, not more than 84, not more than 83, not more than 82, not more than 81, not more than 80, not more than 79, not more than 78, not more than 76, not more than 75, or not more than 74) contiguous amino acid residues of the wild-type protein encoded by BNLF2b gene.

15. The isolated polypeptide or variant thereof according to any one of claims 1 to 14, wherein the isolated polypeptide or variant thereof is attached to a surface of a solid support, or has a modifying group capable of being attached to a solid support;
preferably, the modifying group is biotin or avidin;
preferably, the solid support is selected from the group consisting of magnetic bead or microtiter plate (e.g., microwell plate or ELISA plate).

16. The isolated polypeptide or variant thereof according to any one of claims 1 to 14, wherein the isolated polypeptide or variant thereof bears a detectable label;
preferably, the detectable label is selected from the group consisting of enzyme (e.g., horseradish peroxidase or alkaline phosphatase), chemiluminescent reagent (e.g., acridine ester compound), fluorescent dye or biotin.

17. An isolated nucleic acid molecule, which comprises a nucleotide sequence encoding the isolated polypeptide or variant thereof according to any one of claims 1 to 14.

18. A vector, which comprises the isolated nucleic acid molecule according to claim 17.

19. A host cell, which comprises the isolated nucleic acid molecule according to claim 17 or the vector according to claim 18.

20. A kit, which comprises a capture reagent, in which the capture reagent is selected from the isolated polypeptide or variant thereof according to any one of claims 1 to 14;
preferably, the kit further comprises an instruction for using the isolated polypeptide or variant thereof as the capture reagent to detect a level of an antibody specific to BNLF2b gene-encoded protein in a sample, optionally determining whether the subject has nasopharyngeal cancer or is at risk for nasopharyngeal cancer;
preferably, the capture reagent is selected from the isolated polypeptide or variant thereof according to claim 15;
preferably, the subject is a mammal, such as a human.

21. The kit according to claim 20, which further comprises a detection reagent, wherein the detection reagent is selected from the isolated polypeptide or variant thereof according to claim 16.

22. The kit according to claim 20, which further comprises a detection reagent, wherein the detection reagent is selected from a secondary antibody bearing a detectable label;
preferably, the detectable label is selected from the group consisting of enzyme (e.g., horseradish peroxidase or alkaline phosphatase), chemiluminescent reagent (e.g., acridine ester compound), fluorescent dye or biotin;
preferably, the secondary antibody is specific to an antibody of a species (e.g., human) from which the antibody to be detected is derived;
preferably, the secondary antibody is an anti-immunoglobulin antibody;
preferably, the anti-immunoglobulin antibody is selected from the group consisting of anti-IgG antibody, anti-IgM antibody, and anti-IgA antibody.

23. The kit according to any one of claims 20 to 22, wherein the kit further comprises one or more reagents or devices selected from the group consisting of: (i) a device for collecting or storing the sample (e.g., a blood collection device); (ii) an additional reagent (e.g., buffer, diluent, blocking solution, and/or standard) required to perform the detection.

24. A method for determining whether a subject has nasopharyngeal cancer or is at risk for nasopharyngeal cancer, which comprises:
(1) determining a level of an antibody specific to BNLF2b gene-encoded protein in a sample from the subject; and,
(2) comparing the level to a reference value;
preferably, if the level is higher than the reference value, it is considered that the subject has nasopharyngeal cancer or is at risk for nasopharyngeal cancer;
preferably, the level of the antibody specific to BNLF2b gene-encoded protein in the sample is determined by an immunological assay;
preferably, the immunological assay is selected from the group consisting of enzyme immunoassay (e.g., ELISA), chemiluminescence immunoassay, fluorescent immunoassay or radioimmunoassay.

25. The method according to claim 24, wherein the assay comprises using the isolated polypeptide or variant thereof according to any one of claims 1 to 14 as a capture reagent;
preferably, the capture reagent is selected from the isolated polypeptide or variant thereof according to claim 15.

26. The method according to claim 25, wherein step (1) comprises the steps of:
(1a) contacting a sample from the subject with a capture reagent to obtain an antigen-antibody immune complex; the capture reagent is selected from the isolated polypeptide or variant thereof according to any one of claims 1 to 14;
(1b) determining an amount of the antigen-antibody immune complex obtained in step (1a);
preferably, in step (1b), the amount of the immune complex is determined by an immunological assay;
preferably, the immunological assay is selected from the group consisting of enzyme immunoassay (e.g., ELISA), chemiluminescence immunoassay, fluorescent immunoassay or radioimmunoassay;
preferably, the capture reagent is selected from the isolated polypeptide or variant thereof according to claim 15.

27. The method according to claim 26, wherein, in step (1b), a detection reagent is used to detect the amount of the immune complex, and the detection reagent is selected from the isolated polypeptide or variant thereof according to claim 16.

28. The method according to claim 26, wherein, in step (1b), a detection reagent is used to detect the amount of the immune complex, and the detection reagent is selected from a secondary antibody bearing a detectable label;
preferably, the detectable label is selected from the group consisting of enzyme (e.g., horseradish peroxidase or alkaline phosphatase), chemiluminescent reagent (e.g., acridine ester compound), fluorescent dye or biotin;
preferably, the secondary antibody is specific to an antibody of a species (e.g., human) from which the antibody to be detected is derived;
preferably, the secondary antibody is an anti-immunoglobulin antibody;
preferably, the anti-immunoglobulin antibody is selected from the group consisting of anti-IgG antibody, anti-IgM antibody or anti-IgA antibody; preferably, the anti-immunoglobulin antibody is anti-IgG antibody.

29. The method according to any one of claims 24 to 28, wherein the sample is a blood sample, such as whole blood, plasma or serum.

30. The method according to any one of claims 24 to 29, wherein the subject is a human.

31. The method according to any one of claims 24 to 30, which further comprises:
prior to step (1), providing a sample from the subject; and/or
after step (2), administering to a subject who is considered to have nasopharyngeal cancer or be at risk for nasopharyngeal cancer a therapeutically effective amount of an anti-tumor therapy (e.g., chemotherapy, radiation therapy, and/or immunotherapy) capable of treating nasopharyngeal cancer.

32. Use of a reagent capable of determining a level of an antibody specific to BNLF2b gene-encoded protein in the manufacture of a kit, wherein the kit is used for determining whether a subject has nasopharyngeal cancer or is at risk for nasopharyngeal cancer;
preferably, the reagent is capable of determining the level of the antibody specific to BNLF2b gene-encoded protein by an immunological assay; preferably, the immunological assay is selected from the group consisting of enzyme immunoassay (e.g., ELISA), chemiluminescence immunoassay, fluorescence immunoassay or radioimmunoassay;
preferably, the subject is a human;
preferably, the sample is a blood sample, such as whole blood, plasma or serum;
preferably, the kit determines whether the subject has nasopharyngeal cancer or is at risk for nasopharyngeal cancer by the method according to claim 24.

33. The use according to claim 32, wherein the reagent capable of determining the level of the antibody specific to BNLF2b gene-encoded protein comprises a capture reagent selected from the isolated polypeptide or variant thereof according to any one of claims 1 to 14;
preferably, the capture reagent is selected from the isolated polypeptide or variant thereof according to claim 15;
preferably, the kit determines whether the subject has nasopharyngeal cancer or is at risk for nasopharyngeal cancer by the method according to claim 25 or 26.

34. The use according to claim 33, wherein the reagent capable of determining the level of the antibody specific to BNLF2b gene-encoded protein further comprises a detection reagent, and the detection reagent is selected from the isolated polypeptides or variant thereof according to 16;
preferably, the kit determines whether the subject has nasopharyngeal cancer or is at risk for nasopharyngeal cancer by the method according to claim 27.

35. The use according to claim 33, wherein the reagent capable of determining the level of the antibody specific to BNLF2b gene-encoded protein further comprises a detection reagent, and the detection reagent is selected from a secondary antibody bearing a detectable label;
preferably, the detectable label is selected from the group consisting of enzyme (e.g., horseradish peroxidase or alkaline phosphatase), chemiluminescent reagent (e.g., acridine ester compound), fluorescent dye or biotin;
preferably, the secondary antibody is specific to the antibody of the species (e.g., human) from which the antibody to be detected is derived;
preferably, the secondary antibody is an anti-immunoglobulin antibody;
preferably, the anti-immunoglobulin antibody is selected from the group consisting of anti-IgG antibody, anti-IgM antibody or anti-IgA antibody; preferably, the anti-immunoglobulin antibody is anti-IgG antibody;
preferably, the kit determines whether the subject has nasopharyngeal cancer or is at risk for nasopharyngeal cancer by the method according to claim 28.

36. A method for detecting an antibody specific to BNLF2b gene-encoded protein in a sample, comprising the steps of:
(1) contacting the sample with a capture reagent to obtain an antigen-antibody immune complex; wherein the capture reagent is selected from the isolated polypeptide or variant thereof according to any one of claims 1 to 14;
(2) determining an amount of the antigen-antibody immune complex obtained in step (1);
preferably, in step (2), the amount of the immune complex is determined by an immunological assay;
preferably, the immunological assay is selected from the group consisting of enzyme immunoassay (e.g., ELISA), chemiluminescence immunoassay, fluorescent immunoassay or radioimmunoassay;
preferably, the capture reagent is selected from the isolated polypeptide or variant thereof according to claim 15.

37. The method according to claim 36, wherein, in step (2), a detection reagent is used to determine the amount of the immune complex, and the detection reagent is selected from the isolated polypeptide or variant thereof according to claim 16.

38. The method according to claim 36, wherein, in step (2), a detection reagent is used to determine the amount of the immune complex, and the detection reagent is selected from a secondary antibody bearing a detectable label;
preferably, the detectable label is selected from the group consisting of enzyme (e.g., horseradish peroxidase or alkaline phosphatase), chemiluminescent reagent (e.g., acridine ester compound), fluorescent dye or biotin;
preferably, the secondary antibody is specific to an antibody of a species (e.g., human) from which the antibody to be detected is derived;
preferably, the secondary antibody is an anti-immunoglobulin antibody;
preferably, the anti-immunoglobulin antibody is selected from the group consisting of anti-IgG antibody, anti-IgM antibody or anti-IgA antibody; preferably, the anti-immunoglobulin antibody is anti-IgG antibody.

39. Use of the isolated polypeptide or variant thereof according to any one of claims 1 to 16 in the manufacture of a kit, wherein the kit is used for detecting an antibody specific to BNLF2b gene-encoded protein in a sample.
